# EUROPEAN PATENT APPLICATION

(11) **EP 2 468 279 A1**
(43) Date of publication of application: **27.06.2012**
(21) Application number: 10306479.6
(22) Date of filing: 21.12.2010
(51) Int. Cl.: A61K 31/4402, A61K 31/443, A61K 31/4436, A61P 9/00, A61P 25/28, A61P 1/00, A61P 43/00

(54) **Prokineticin 1 receptor agonists and their uses**

(71) Applicant: Université de Strasbourg, 67000 Strasbourg (FR); Centre National de la Recherche Scientifique, 75016 Paris (FR)
(72) Inventor: The designation of the inventor has not yet been filed
(74) Representative: Gallois, Valérie

(57) **Abstract**

The present invention discloses non peptide prokineticin 1 receptor agonists and their uses for the treatment of PKR1 mediated disorders, in particular for the treatment of vascular diseases, neurodegenerative diseases, diseases involving impaired gastrointestinal motility, obesity, Kallmann syndrome, normosmic hypogonadotropic hypogonadism and disturbances of circadian rhythm, and to prevent or limit the toxicity, in particular the cardiotoxicity and neurotoxicity, of drugs.

## Description

### Field of the Invention

The present invention relates to non peptide prokineticin receptor-1 (PKR1) agonists and pharmaceutical compositions comprising thereof. The present invention also relates to the use of non peptide PKR1 agonists for the treatment of PKR1 mediated disorders and to prevent or limit the toxicity, in particular the cardiotoxicity and the neurotoxicity, of drugs.

### Background of the Invention

Prokineticin-1, also called endocrine gland-derived vascular endothelial growth factor (EG-VEGF) and prokineticin-2, also called Bv8, are two secreted proteins from the AVIT secreted protein family that are widely express among mammalian tissues.

These molecules exert their biological functions through activation of two closely related G-protein-coupled receptors, the prokineticin receptors 1 and 2 (PKR1 and PKR2) (Lin et al., 2002; Masuda et al., 2002; Soga et al., 2002). Both PKR1 and PKR2 are highly expressed in human and rat endocrine tissues, including thyroid, pituitary, and adrenal glands, as well as in the ovary and testis. PKR2 and to a lesser extent PKR1 are expressed in the brain. PKR1, originally called GPR73, is mainly expressed in spleen, prostrate, pancreas, monocytes leukocytes, and in human heart (Urayama et al., 2007 and Parker et al., 2000).

Prokineticins exhibit different affinities for their receptors, prokineticin-2 being the most potent agonist for both receptors (Chen et al. 2005). PKR1 and PKR2 share 85% amino acid identity and diverge mainly in their N-terminal sequences. PKR2 is encoded by a gene located on human chromosome region 2q14, whereas the PKR2 gene is located on 2p13 (Parker etal., 2000).

Prokineticins are involved in regulating various biological processes that include gastrointestinal motility (Li et al., 2001), pain sensitization (Negri et al., 2005), angiogenesis (LeCouter et al., 2001; LeCouter et al., 2003), circadian rhythms (Cheng et al., 2002), olfactory bulb activation (Ng et al., 2005), hematopoiesis (LeCouter et al., 2004), monocyte differentiation (Dorsch et al., 2005), and macrophage activation (Martucci et al., 2006). In cardiomyocytes, prokineticin-2 via PKR1 protects cardiomyocytes against hypoxia induced apoptosis (Urayama et al., 2007). Moreover, transient PKR1 gene transfer reduces mortality and preserves left ventricular function by promoting angiogenesis and cardiomyocyte survival after the coronary ligation as a mouse model of myocardial infarction (Urayama et al., 2007).

Recent studies have revealed that prokineticins and their receptors are associated with the pathologies of various human diseases and genetic syndromes including, for example, heart failure (Urayama et al. 2007), abdominal aortic aneurysm (Choke et al. 2009) and Kallmann syndrome (Hardelin and Dodé 2008), and familial hyperthyroidism (Warner et al., 2006).

A designed PK2β peptide possessing only 47 amino acids of the N-terminus of PK2 has been described as a potent agonist for PKR1 (WO2005/097826). However, the use of peptide drugs is limited due to the instability (proteolytic cleavage of the peptide backbone) and the low bioavailability (poor membrane transport capability) of these molecules. Furthermore, peptide drugs have usually to be administered by the parenteral route because of insufficient absorption from the gastrointestinal tract.

Consequently, there is a strong need of developing non peptide agonists for PKR1 that could be administered by the oral route to treat PKR1 mediated disorders.

### Summary of the Invention

The object of the present invention is to provide non peptide prokineticin receptor-1 I (PKR2) agonists.

In a first aspect, the present invention provides a pharmaceutical composition comprising a PKR2 agonist of formula (I) 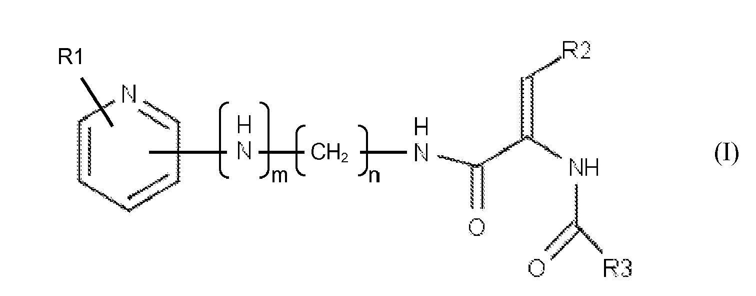 wherein
m is selected from 0 and 1;
n is selected from 0, 1 and 2;
R¹ is selected from the group consisting of a hydrogen atom; a halogen atom; a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; a (C₆-C₁₂)-aryl; a 5 to 7-membered-ring heterocycle; a (C₁-C₆)-alkoxy, a (C₂-C₆)-acyl, a (C₂-C₆)-ester, a (C₁-C₆)-amine, a (C₁-C₆)-amide, a (C₁-C₆)-imine, a (C₁-C₆)-nitrile, a (C₁-C₆)-thioalkyl, a (C₁-C₆)-sulfone and a (C₁-C₆)-sulfoxide group wherein the alkyl part of the group is optionally interrupted by one of several heteroatoms chosen among N, O and S, and is optionally substituted by at least one substituent selected from a hydroxyl, a carboxylic group, an aldehyde, a halogen atom and a thiol;
R² is selected from the group consisting of a (C₆-C₁₂)-aryl group and a 5 to 7-membered-ring heterocycle, preferably from phenyl and furan, optionally substituted by at least one substituent selected from the group consisting of a (C₁-C₆)-alkyl, a (C₂-C₆)-alkenyl, a (C₂-C₆)-alkynyl, a (C₆-C₁₂)-aryl, a 5 to 7-membered-ring heterocycle, a (C₁-C₆)-alkoxy, a (C₂-C₆)-acyl, a (C₁-C₆)-alcohol, a carboxylic group, a (C₂-C₆)-ester, a (C₁-C₆)-amine, an amino group, a (C₁-C₆)-amide, a (C₁-C₆)-imine, a (C₁-C₆)-nitrile, a hydroxyl, an aldehyde, a (C₁-C₆)-halogenoalkyl, a thiol, a (C₁-C₆)-thioalkyl, a (C₁-C₆)-sulfone, a (C₁-C₆)-sulfoxide group and a halogen atom;
R³ is selected from the group consisting of a (C₁-C₆)-alkyl, a (C₂-C₆)-alkenyl, a (C₂-C₆)-alkynyl and a (C₁-C₆)-amine group, optionally substituted by at least one substituent selected from a hydroxyl, a carboxylic group, an aldehyde, a halogen atom and a thiol; and a (C₆-C₁₂)-aryl and a 5 to 7-membered-ring heterocycle, optionally substituted by at least one substituent selected from the group consisting of a (C₁-C₆)-alkyl, a (C₁-C₆)-alkoxy group and a halogen atom;
or any pharmaceutically acceptable salt thereof.

The present invention also concerns a PKR1 agonist as described above as a medicament.

The present invention further concerns a PKR1 agonist as described above as a cardioprotective agent.

The present invention further concerns a PKR1 agonist as described above as a neuroprotective agent.

In another aspect, the present invention concerns a pharmaceutical composition comprising a PKR1 agonist as described above and a pharmaceutically acceptable carrier and/or excipient. The pharmaceutical composition according to the invention can further comprise one or more other active compounds associated with pharmaceutically acceptable excipients and/or carriers.

In another aspect, the present invention concerns a pharmaceutical composition or a PKR1 agonist as described above for use for the prevention or treatment of a disease or a disorder selected from the group consisting of a vascular disease, a neurodegenerative disease, a disease involving impaired gastrointestinal motility, obesity, Kallmann syndrome, normosmic hypogonadotropic hypogonadism and disturbances of circadian rhythm.

In a further aspect, the present invention concerns a pharmaceutical composition or a PKR1 agonist as described above for use to prevent or to limit the neurotoxicity and/or cardiotoxicity of a compound, in particular of an antiviral or antineoplastic agent.

In last aspect, the present invention concerns a product containing a PKR1 agonist of the invention and an antineoplastic agent, as a combined preparation for simultaneous, separate or sequential use in the treatment of cancer.

### Legends of the Figures

**Figure 1****:** Formulas of PKR1 agonists IS1 to IS12 and IS14 to IS19.
**Figure 2****:** Synthesis of the PKR1 agonists of the invention.
**Figure 3****:** Induction of *in vitro* angiogenesis by prokineticin-2 and IS1 compound. ***Fig. 3A***: tube-like formation of the endothelial cells on Matrigel. ***Fig. 3B***: quantification of the tube-like formation of the endothelial cells on Matrigel.
**Figure 4****:** Induction of *in vitro* angiogenesis by prokineticin-2 and IS compounds. H5V cells were infected with adenovirus carrying PKR1 cDNA in order to dominantly express PKR1. ***Fig. 4A******:*** 1 µM prokineticin-2 or IS compound. ***Fig. 4B****:* 10 µM prokineticin-2 or IS compound.
**Figure 5****:** intracellular calcium release detected by fura-4 labeled CHO utilizing confocal microscope analyses.
**Figure 6****:** PKR1 internalization in CHO cells GFP-PKR1.

### Detailed description of the invention

The inventors disclose herein non peptide prokineticin receptor-1 (PKR1) agonists.

They demonstrated that these agonists are able to activate the PKR1 signalling pathway by specific binding on PKR1.

### Definitions

"Prokineticin receptor 1" or "PKR2", as used herein, refers to a G-protein-coupled receptor that could be activated by the binding of prokineticin-1 or prokineticin-2. It may be also named GPR73 or PROKR1. The reference amino acid sequence for human PKR1 can be retrieved from the Genbank Accession No. NP_620414. The reference entry for human PKR1 in the transcriptome database UniGene is Hs.683430.

As used herein, the term "alkyl" refers to a univalent radical containing only carbon and hydrogen atoms arranged in a chain. The alkyl group may be linear or branched. More specifically, the term "alkyl" means a group such as methyl, ethyl, propyl, isopropyl, butyl, isobutyl, tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl and the other isomeric forms thereof. (C₁-C₆)-alkyl includes methyl, ethyl, propyl, isopropyl, butyl, isobutyl, tert-butyl, pentyl, hexyl and the other isomeric forms thereof. (C₁-C₃)-alkyl includes methyl, ethyl, propyl, or isopropyl. The term "Me" as used herein, refers to a methyl group. The term "Et" as used herein, refers to an ethyl group.

As used herein, the term "alkoxy" corresponds to an alkyl group bonded to the molecule by an -O- (ether) bond. (C₁-C₆)- alkoxy includes methoxy, ethoxy, propyloxy, butyloxy, pentyloxy, hexyloxy and the other isomeric forms thereof. (C₁-C₃)-alkoxy includes methoxy, ethoxy, propyloxy, and isopropyloxy.

The term "alkenyl" refers to an alkyl group having at least one unsaturated ethylene bond and the term "alkynyl" refers to an alkyl group having at least one unsaturated acetylene bond. (C₂-C₆)-alkenyl includes ethenyl, propenyl (1-propenyl or 2-propenyl), 1-or 2- methylpropenyl, butenyl (1-butenyl, 2-butenyl, or 3-butenyl), methylbutenyl, 2-ethylpropenyl, pentenyl (1-pentenyl, 2-pentenyl, 3-pentenyl, 4-pentenyl), hexenyl (1-hexenyl, 2-hexenyl, 3-hexenyl, 4-hexenyl, 5-hexenyl), and the other isomeric forms thereof. (C₂-C₃)-alkenyl includes ethenyl, 1-propenyl and 2-propenyl. (C₂-C₆)-alkynyl includes ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl and the other isomeric forms thereof. (C₂-C₃)-alkynyl includes ethynyl, 1-propynyl and 2-propynyl.

The "aryl" groups are monocyclic, bicyclic, or polycyclic aromatic hydrocarbons. Examples include phenyl, biphenyl, α-naphthyl, β-naphthyl, anthracenyl, phenanthrenyl, fluorenyl, indenyl, pentalenyl, azulenyl, biphenylenyl and the like. This term is also intended to include the partially hydrogenated derivatives of the aromatic hydrocarbons groups enumerated above. The aryl groups of the present invention can be substituted or unsubstituted. (C₆-C₁₂)-aryl includes phenyl, biphenyl, indenyl, pentalenyl and azulenyl.

The term "heterocycle", as used herein, refers to 5-10 membered heterocyclic ring systems comprising one or more heteroatoms, preferably 1 to 5 endocyclic heteroatoms. They may be mono-, bi- or tri-cyclic systems. They may be aromatic or not. Examples of aromatic heterocycles include pyridine, pyridazine, pyrimidine, pyrazine, furan, thiophene, pyrrole, oxazole, thiazole, isothiazole, imidazole, pyrazole, oxadiazole, triazole, thiadiazole and triazine groups. Examples of non-aromatic heterocycles include piperazine and piperidine. Examples of 5 to 7-membered-ring heterocycles include pyrrolidine, tetrahydrofuran, tetrahydrothiophene, pyrrole, furan, thiophene, piperidine, tetrahydropyran, thiane, pyridine, pyrylium, thiopyran, azepane, oxepane, thiepane, azepine, oxepine and thiepine.

The term "heteroatom" refers to any atom that is not carbon or hydrogen. In particular embodiments, this term refers to N, S, or O.

"Acyl" groups correspond to alkyl groups bonded to the molecule by a -CO-(carbonyl) group. (C₂-C₆)-acyl includes acetyl, propylacyl, butylacyl, pentylacyl, hexylacyl and the other isomeric forms thereof. (C₂-C₃)-acyl includes acetyl, propylacyl and isopropylacyl.

"Alcohol" groups correspond to alkyl groups containing at least one hydroxyl group. Alcohol can be primary, secondary or tertiary. (C₁-C₆)-alcohol includes methanol, ethanol, propanol, butanol, pentanol, hexanol and the other isomeric forms thereof. (C₁-C₃)-alcohol includes methanol, ethanol, propanol and isopropanol. 1.

"Ester" groups correspond to alkyl groups bonded to the molecule by a -COO- (ester) bond. (C₂-C₆)-ester includes methylester, ethylester, propylester, butylester, pentylester and the other isomeric forms thereof. (C₂-C₃)-ester includes methylester and ethylester.

"Amine" groups correspond to alkyl groups bonded to the molecule by a -N- (amine) bond. (C₁-C₆)-amine includes methylamine, ethylamine, propylamine, butylamine, pentylamine, hexylamine and the other isomeric forms thereof. (C₁-C₃)-amine includes methylamine, ethylamine, and propylamine.

"Amide" groups correspond to alkyl groups containing at least one carbonyl (CO) group bound to a nitrogen atom. (C₁-C₆)-amide includes methylamide, ehtylamide, propylamide, butylamide, pentylamide, hexylamide and the other isomeric forms thereof. (C₁-C₃)-amide includes methylamide, ehtylamide and propylamide.

"Imine" groups correspond to alkyl groups having a (-C=N-) bond. (C₁-C₆)-imine includes methylimine, ethylimine, propylimine, butylimine, pentylimine, hexylimine and the other isomeric forms thereof. (C₁-C₃)-imine includes methylimine, ethylimine, and propylimine.

"Nitrile" groups correspond to alkyl groups having a (-C≡N-) bond.

The halogen can be Cl, Br, I, or F, preferably Cl, Br or F, more preferably Cl.

"Halogenoalkyl" groups correspond to alkyl groups having at least one halogen. The groups can be monohalogenated or polyhalogenated, containing the same or different halogen atoms. For example, the group can be an trifluoroalkyl (CF₃-R). (C₁-C₆)-halogenoalkyl includes halogenomethyl, halogenoethyl, halogenopropyl, halogenobutyl, halogenopentyl, halogenohexyl and the other isomeric forms thereof. (C₁-C₃)-halogenoalkyl includes halogenomethyl, halogenoethyl, and halogenopropyl.

"Thioalkyl" groups correspond to alkyl groups bonded to the molecule by a -S-(thioether) bond. (C₁-C₆)-thioalkyl includes thiomethyl, thioethyl, thiopropyl, thiobutyl, thiopentyl, thiohexyl and the other isomeric forms thereof. (C₁-C₃)-thioalkyl includes thiomethyl, thioethyl, and thiopropyl.

"Sulfone" groups correspond to alkyl groups bonded to the molecule by a -SOO-(sulfone) bond. (C₁-C₆)-sulfone includes methylsulfone, ethylsulfone, propylsulfone, butylsulfone, pentylsulfone, hexylsulfone and the other isomeric forms thereof. (C₁-C₃)-sulfone includes methylsulfone, ethylsulfone and propylsulfone.

"Sulfoxide" groups correspond to alkyl groups bonded to the molecule by a -SO-(sulfoxide) group. (C₁-C₆) sulfoxide includes methylsulfoxide, ethylsulfoxide, propylsulfoxide, butylsulfoxide, pentylsulfoxide, hexylsulfoxide and the other isomeric forms thereof. (C₁-C₃) sulfoxide includes methylsulfoxide, ethylsulfoxide, propylsulfoxide and isopropylsulfoxide.

The term "pharmaceutically acceptable salt" refers to salts which are non-toxic for a patient and suitable for maintaining the stability of a therapeutic agent and allowing the delivery of said agent to target cells or tissue. Pharmaceutically acceptable salts are well known in the art (Berge et al., 1977). These salts can be prepared in situ during the final isolation and purification of the agonists of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, 3-phenylpropionate, phosphate, picrate, pivalate, propionate, stearate, succinate, sulfate, tartrate, thiocyanate, p-toluenesulfonate, undecanoate, valerate salts, and the like.

As used herein, the term "patient" or "subject" refers to any mammal, preferably a human being.

As used herein, the term "neuroprotective agent" refers to an agent that protects neuronal cells from damages, in particular from cell death.

As used herein, the term "cardioprotective agent" refers to an agent that protects cardiomyocytes from damages, in particular from cell death.

As used herein, the term "antineoplastic agent" refers to an agent with anti-cancer activity that inhibits or halts the growth of cancerous cells or immature pre-cancerous cells, kills cancerous cells or immature pre-cancerous cells, increases the susceptibility of cancerous or pre-cancerous cells to other antineoplastic agents, and/or inhibits metastasis of cancerous cells. These agents may include chemical agents as well as biological agents.

As used herein, the term "prevention or treatment of a disease" or "prevention or treatment of a disorder" or "to prevent or to treat a disease or "to prevent or to treat a disorder" refers to any act intended to ameliorate the health status of patients such as therapy, prevention, prophylaxis and retardation of the disease or the disorder. In certain embodiments, such term refers to the amelioration or eradication of the disease or the disorder, or symptoms associated with said disease or disorder. In other embodiments, this term refers to minimizing the spread or worsening of the disease or disorder resulting from the administration of one or more therapeutic agents to a subject with said disease or disorder.

### PKR1 agonists

In a first aspect, the present invention discloses a PKR1 agonist of formula (I) 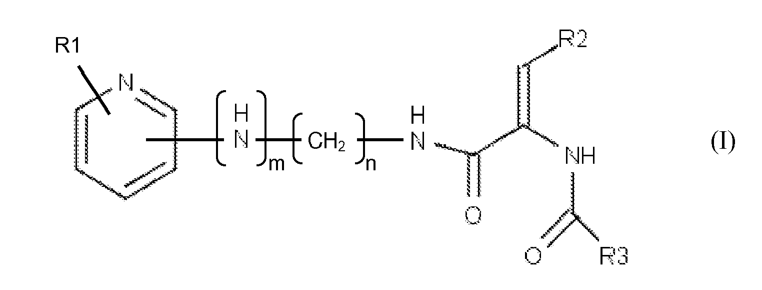 wherein
m is selected from 0 and 1;
n is selected from 0, 1 and 2;
R¹ is selected from the group consisting of a hydrogen atom; a halogen atom; a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; a (C₆-C₁₂)-aryl; a 5 to 7-membered-ring heterocycle; a (C₁-C₆)-alkoxy, a (C₂-C₆)-acyl, a (C₂-C₆)-ester, a (C₁-C₆)-amine, a (C₁-C₆)-amide, a (C₁-C₆)-imine, a (C₁-C₆)-nitrile, a (C₁-C₆)-thioalkyl, a (C₁-C₆)-sulfone and a (C₁-C₆)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a (C₆-C₁₂)-aryl group and a 5 to 7-membered-ring heterocycle, preferably from phenyl and furan, optionally substituted by at least one substituent selected from the group consisting of a (C₁-C₆)-alkyl, a (C₂-C₆)-alkenyl, a (C₂-C₆)-alkynyl, a (C₆-C₁₂)-aryl, a 5 to 7-membered-ring heterocycle, a (C₁-C₆)-alkoxy, a (C₂-C₆)-acyl, a (C₁-C₆)-alcohol, a carboxylic group, a (C₂-C₆)-ester, a (C₁-C₆)-amine, an amino group, a (C₁-C₆)-amide, a (C₁-C₆)-imine, a (C₁-C₆)-nitrile, a hydroxyl, an aldehyde, a (C₁-C₆)-halogenoalkyl, a thiol, a (C₁-C₆)-thioalkyl, a (C₁-C₆)-sulfone, a (C₁-C₆)-sulfoxide group and a halogen atom;
R³ is selected from the group consisting of a (C₁-C₆)-alkyl, a (C₂-C₆)-alkenyl, a (C₂-C₆)-alkynyl and a (C₁-C₆)-amine group, optionally substituted by at least one substituent selected from a hydroxyl, a carboxylic group, an aldehyde, a halogen atom and a thiol; and a (C₆-C₁₂)-aryl and a 5 to 7-membered-ring heterocycle, optionally substituted by at least one substituent selected from the group consisting of a (C₁-C₆)-alkyl, a (C₁-C₆)-alkoxy group and a halogen atom;
or any pharmaceutically acceptable salt thereof.

In particular, the agonist of formula (I) is defined as above, with the proviso that when m is 0, n is 1, R¹ is a hydrogen atom and R² is a furan group, then R³ is not a phenyl group or a phenyl group substituted in para by a methoxy group; and when m is 0, n is 0, R¹ is a hydrogen atom and R² is a furan group, then R³ is not a phenyl group.

In an embodiment,
R¹ is selected from the group consisting of a hydrogen atom; a halogen atom; a (C₁-C₃)-alkyl, a (C₂-C₃)-alkenyl and a (C₂-C₃)-alkynyl group, optionally substituted by at least one substituent selected from a hydroxyl, a carboxylic group, an aldehyde, a halogen atom and a thiol; a (C₆-C₁₂)-aryl, preferably a phenyl group; a 5 to 7-membered-ring heterocycle; a (C₁-C₃)-alkoxy, a (C₂-C₃)-acyl, a (C₂-C₃)-ester, a (C₁-C₃)-amine, a (C₁-C₃)-amide, a (C₁-C₃)-imine, a (C₁-C₃)-nitrile, a (C₁-C₃)-thioalkyl, a (C₁-C₃)-sulfone and a (C₁-C₃)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a phenyl group and a 5 to 7-membered-ring N-, O- or S-heterocycle, optionally substituted by at least one substituent selected from the group consisting of a (C₁-C₃)-alkyl, a (C₂-C₃)-alkenyl, a (C₂-C₃)-alkynyl, a phenyl group, a 5-membered-ring heterocycle, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a (C₁-C₃)-imine, a (C₁-C₃)-nitrile, a hydroxyl, an aldehyde, a (C₁-C₃)-halogenoalkyl, a thiol, a (C₁-C₃)-thioalkyl, a (C₁-C₃)-sulfone, a (C₁-C₃)-sulfoxide group and a halogen atom;
R³ is selected from the group consisting of a (C₁-C₆)-alkyl, a (C₂-C₃)-alkenyl, a (C₁-C3)-amine and a (C₂-C₃)-alkynyl group, optionally substituted by at least one substituent selected from a hydroxyl, a carboxylic group, an aldehyde, a halogen atom and a thiol; and a phenyl group and a 5 to 7-membered-ring heterocycle, optionally substituted by at least one substituent selected from the group consisting of a (C₁-C₃)-alkyl, a (C₁-C₃)-alkoxy group and a halogen atom.

In an embodiment, R¹ is selected from the group consisting of a hydrogen atom; a halogen atom; a (C₁-C₃)-alkyl, optionally substituted by at least one substituent selected from a hydroxyl, a carboxylic group, an aldehyde, a halogen atom and a thiol; a (C₁-C₃)-alkoxy, a (C₂-C₃)-acyl, a (C₂-C₃)-ester, a (C₁-C₃)-amine, a (C₁-C₃)-amide and a (C₁-C₃)-thioalkyl, group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol.

In a particular embodiment, R¹ is selected from the group consisting of a hydrogen atom, a halogen atom, a (C₁-C₃)-alkyl group and a (C₁-C₃)-alkoxy group, said (C₁-C₃)-alkyl group and/or (C₁-C₃)-alkoxy group being optionally substituted by at least one substituent selected from an hydroxyl, a carboxylic group, an aldehyde, a halogen atom and a thiol.

In a more particular embodiment, R¹ is selected from the group consisting of a hydrogen atom, a halogen atom, a (C₁-C₃)-alkyl group and a (C₁-C₃)-alkoxy group. Preferably, R¹ is selected from the group consisting of a hydrogen atom, a halogen atom, a methyl group and a methoxy group. More preferably, R¹ is a hydrogen atom.

In an embodiment, R² is selected from the group consisting of a phenyl group and a 5 to 7-membered-ring N-, O- or S-heterocycle, preferably a 5-membered-ring N-, O- or S-heterocycle, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom.

In a particular embodiment, R² is selected from the group consisting of a phenyl, a pyrrole, a furan and a thiophene group, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom.

In a more particular embodiment, R² is selected from the group consisting of a phenyl, a pyrrole, a furan and a thiophene group, optionally substituted by at least one substituent selected from the group consisting of a (C₁-C₃)-alkyl and a (C₁-C₃)-alkoxy group, preferably methyl and methoxy. Preferably, R² is selected from the group consisting of a phenyl group, optionally substituted by a methoxy, a furan and a thiophene group.

In an embodiment, R³ is selected from the group consisting of a (C₁-C₆)-alkyl and a (C₁-C₆)-amine group optionally substituted by at least one substituent selected from a hydroxyl, a carboxylic group, an aldehyde, a halogen atom and a thiol; and a phenyl group and a 5 to 7-membered-ring N-, O- or S-heterocycle, optionally substituted by at least one substituent selected from the group consisting of a (C₁-C₃)-alkyl, a (C₁-C₃)-alkoxy group and a halogen atom.

In a particular embodiment, R³ is selected from the group consisting of a methyl, an ethyl, a propyl, an isopropyl, a butyl, an isobutyl, a tert-butyl group; a phenyl, a pyrrole, a furan and a thiophene group, optionally substituted by at least one substituent selected from the group consisting of a (C₁-C₃)-alkyl group, preferably methyl, a (C₁-C₃)-alkoxy group, preferably methoxy, and a halogen atom, preferably chlorine.

In a more particular embodiment, R³ is selected from the group consisting of a methyl, an ethyl, a propyl, an isopropyl, a butyl, an isobutyl, a tert-butyl group; and a phenyl group, optionally substituted by at least one substituent selected from the group consisting of a (C₁-C₃)-alkyl group, preferably methyl, a (C₁-C₃)-alkoxy group, preferably methoxy, and a halogen atom, preferably chlorine. Preferably, R³ is selected from the group consisting of a tert-butyl group and a phenyl group, optionally substituted by at least one substituent selected from the group consisting of a methyl group, a methoxy group and chlorine. More preferably, R³ is selected from the group consisting of a tert-butyl, a phenyl, a methoxyphenyl and a chlorophenyl group.

In an embodiment, R¹ is selected from the group consisting of a hydrogen atom; a halogen atom; a (C₁-C₃)-alkyl, optionally substituted by at least one substituent selected from a hydroxyl, a carboxylic group, an aldehyde, a halogen atom and a thiol; a (C₁-C₃)-alkoxy, a (C₂-C₃)-acyl, a (C₂-C₃)-ester, a (C₁-C₃)-amine, a (C₁-C₃)-amide and a (C₁-C₃)-thioalkyl, group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a phenyl group and a 5 to 7-membered-ring N-, O- or S-heterocycle, preferably a 5-membered-ring N-, O- or S-heterocycle, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom; and
R³ is selected from the group consisting of a (C₁-C₆)-alkyl and a (C₁-C₆)-amine group, optionally substituted by at least one substituent selected from a hydroxyl, a carboxylic group, an aldehyde, a halogen atom and a thiol; and a phenyl group and a 5 to 7-membered-ring N-, O- or S-heterocycle, optionally substituted by at least one substituent selected from the group consisting of a (C₁-C₃)-alkyl, a (C₁-C₃)-alkoxy group and a halogen atom.

In a particular embodiment,
R¹ is selected from the group consisting of a hydrogen atom, a halogen atom, a (C₁-C₃)-alkyl group and a (C₁-C₃)-alkoxy group, said (C₁-C₃)-alkyl group and/or (C₁-C₃)-alkoxy group being optionally substituted by at least one substituent selected from an hydroxyl, a carboxylic group, an aldehyde, a halogen atom and a thiol;
R² is selected from the group consisting of a phenyl, a pyrrole, a furan and a thiophene group, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom; and
R³ is selected from the group consisting of a methyl, an ethyl, a propyl, an isopropyl, a butyl, an isobutyl, a tert-butyl group; a phenyl, a pyrrole, a furan and a thiophene group, optionally substituted by at least one substituent selected from the group consisting of a (C₁-C₃)-alkyl group, preferably methyl, a (C₁-C₃)-alkoxy group, preferably methoxy, and a halogen atom, preferably chlorine.

In a more particular embodiment,
R¹ is selected from the group consisting of a hydrogen atom, a halogen atom, a (C₁-C₃)-alkyl group and a (C₁-C₃)-alkoxy group, preferably from the group consisting of a hydrogen atom, a halogen atom, a methyl group and a methoxy group, and more preferably is a hydrogen atom;
R² is selected from the group consisting of a phenyl, a pyrrole, a furan and a thiophene group, optionally substituted by at least one substituent selected from the group consisting of a (C₁-C₃)-alkyl and a (C₁-C₃)-alkoxy, preferably from the group consisting of a phenyl group, optionally substituted by a methoxy, a furan and a thiophene group; and
R³ is selected from the group consisting of a methyl, an ethyl, a propyl, an isopropyl, a butyl, an isobutyl, a tert-butyl group; and a phenyl group, optionally substituted by at least one substituent selected from the group consisting of a (C₁-C₃)-alkyl group, a (C₁-C₃)-alkoxy group and a halogen atom, preferably from the group consisting of a tert-butyl, a phenyl, a methoxyphenyl and a chlorophenyl group.

In an embodiment, the PKR1 agonist has the formula (II) 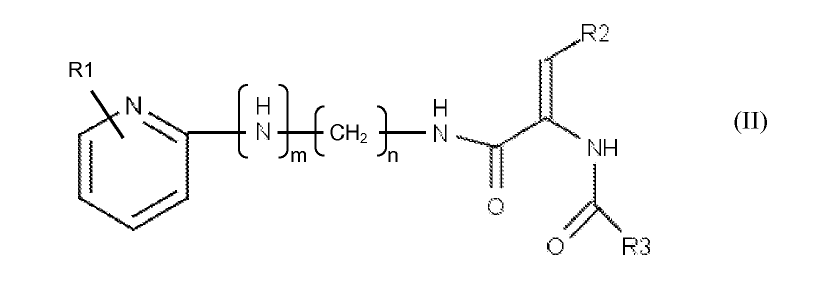 m, n, R¹, R² and R³ being as defined in formula (I) and in the above disclosed embodiments.

In another embodiment, the PKR1 agonist has the formula (III) 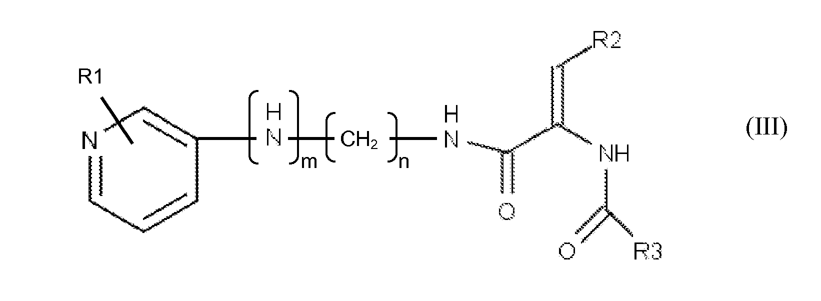 m, n, R¹, R² and R³ being as defined in formula (I) and in the above disclosed embodiments.

In another embodiment, the PKR1 agonist has the formula (IV) 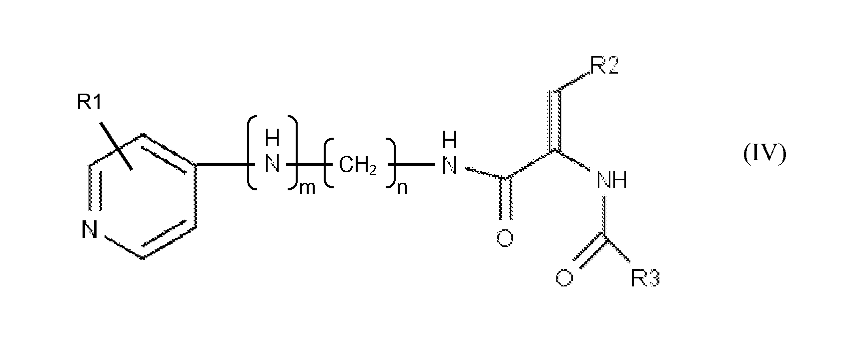 m, n, R¹, R² and R³ being as defined in formula (I) and in the above disclosed embodiments.

In an embodiment, the PKR1 agonist has the formula (I), (II), (III) or (IV) and R¹ is a hydrogen atom; R² is selected from the group consisting of a 5-membered-ring heterocycle and a phenyl group, preferably from a furan, a thiophene and a phenyl group, optionally substituted by a (C₁-C₃)-alkoxy group, preferably a methoxy group; and R³ is selected from the group consisting of a (C₁-C₆)-alkoxy, preferably a tert-butyl group, and a phenyl group, optionally substituted by a substituent selected from the group consisting of a (C₁-C₃)-alkoxy group and a halogen atom, preferably from a methoxy group and chlorine.

In another embodiment, the PKR1 agonist has the formula (I), (II), (III) or (IV), and R¹ is a hydrogen atom; R² is selected from the group consisting of a 5-membered-ring heterocycle and a phenyl group, preferably from a furan, a thiophene and a phenyl group, optionally substituted by a (C₁-C₃)-alkoxy group, preferably a methoxy group; and R³ is a phenyl group. Preferably, in this embodiment, the PKR1 agonist has the formula (II).

In a particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group. Preferably, in this embodiment, the PKR1 agonist has the formula (II).

In another particular embodiment, the PKR1 agonist has the formula (I), (II), (III) or (IV), and m is 1; n is selected from 0 and 2; R¹ is a hydrogen atom; R² is selected from the group consisting of a 5-membered-ring O- or S-heterocycle and a phenyl group, preferably from a furan, a thiophene and a phenyl group, optionally substituted by a (C₁-C₃)-alkoxy group, preferably a methoxy group; and R³ is a phenyl group. Preferably, in this embodiment, the PKR1 agonist has the formula (II).

In another particular embodiment, the PKR1 agonist has the formula (I), (II), (III) or (IV), and m is 1; n is 2; R¹ is a hydrogen atom; R² is selected from the group consisting of a 5-membered-ring O- or S-heterocycle and a phenyl group, preferably from a furan, a thiophene and a phenyl group, optionally substituted by a (C₁-C₃)-alkoxy group, preferably a methoxy group; and R³ is a phenyl group. Preferably, in this embodiment, the PKR1 agonist has the formula (II).

In another embodiment, the PKR1 agonist has the formula (I), (II), (III) or (IV), and m is 0; n is selected from 0, 1 and 2; R¹ is a hydrogen atom; R² is a 5-membered-ring O-heterocycle, preferably a furan group; and R³ is selected from the group consisting of a phenyl and a (C₁-C₆)-alkyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (III).

In a particular embodiment, the PKR1 agonist has the formula (I), (II), (III) or (IV), m is 0; n is selected from 1 and 2; R¹ is a hydrogen atom; R² is a 5-membered-ring O-heterocycle, preferably a furan group; and R³ is selected from the group consisting of a phenyl and a (C₁-C₆)-alkyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group substituted by a (C₁-C₃)-alkoxy group, preferably a methoxy group. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group substituted by a halogen atom, preferably chlorine. Preferably, in this embodiment, the PKR1 agonist has the formula (III).

In another particular embodiment, the PKR1 agonist has the formula (I), (II), (III) or (IV), m is 0; n is selected from 1 and 2; R¹ is a hydrogen atom; R² is a 5-membered-ring O-heterocycle, preferably a furan group; and R³ is a (C₁-C₆)-alkyl group, preferably a tert-butyl group. Preferably, in this embodiment, the PKR1 agonist has the formula (III).

In another embodiment, the PKR1 agonist has the formula (I), (II), (III) or (IV), and m is 0; n is selected from 0 and 1; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (IV).

In a particular embodiment, the PKR1 agonist has the formula (I), (II), (III) or (IV), m is 0; n is 1; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (IV).

In another particular embodiment, the PKR1 agonist has the formula (I), (II), (III) or (IV), and m is 0; n is 1; R¹ is a hydrogen atom; R² is a 5-membered-ring O-heterocycle, preferably a furan group; and R³ is a phenyl group. Preferably, in this embodiment, the PKR1 agonist has the formula (IV).

In another particular embodiment, the PKR1 agonist has the formula (I), (II), (III) or (IV), and m is 0; n is 0; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (IV).

In another particular embodiment, the PKR1 agonist has the formula (I), (II), (III) or (IV), and m is 0; n is 0; R¹ is a hydrogen atom; R² is a 5 to 7-membered-ring O-heterocycle, preferably a furan group; and R³ is a phenyl group. Preferably, in this embodiment, the PKR1 agonist has the formula (IV).

In an embodiment, the PKR1 agonist has the formula (II) or (IV), m is selected from 0 and 1; n is selected from 0, 1 and 2; R¹ is a hydrogen atom; R² is selected from the group consisting of a furan group and a phenyl group, optionally substituted by a methoxy group; and R³ is a phenyl group.

In a particular embodiment, the PKR1 agonist has the formula (II) or (IV), m is selected from 0 and 1; n is selected from 0, 1 and 2; R¹ is a hydrogen atom; R² is selected from the group consisting of a furan group and a phenyl group substituted by a methoxy group; and R³ is a phenyl group.

In an embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group. In another embodiment, the PKR1 agonist of formula (I), (II), (III) or (IV) has one or several of the following features: a) m is 1; b) n is 2; c) R¹ is a hydrogen atom; d) R² is a phenyl group; and e) R³ is a phenyl group. In another embodiment, the PKR1 agonist of formula (I), (II), (III) or (IV) has one or several of the following features: a) m is 1; b) n is 2; c) R¹ is a hydrogen atom; d) R² is a phenyl group substituted by a (C₁-C₃)-alkoxy group, preferably methoxy; and e) R³ is a phenyl group. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group substituted by a halogen atom, preferably chlorine. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group substituted by a (C₁-C₃)-alkoxy group, preferably methoxy. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a (C₁-C₆)-alkyl group, preferably a tert-butyl group. In an embodiment, the PKR1 agonist of formula (IV) has one or several of the following features: a) m is 0; b) n is 1; c) R¹ is a hydrogen atom; d) R² is a furan group; and e) R³ is a phenyl group.

The PKR1 agonist of formula (I), (II), (III) or (IV) may meet one of the above-listed features. It may also meet two of these features, for instance a) and b); a) and c); a) and d); a) and e); b) and c); b) and d); b) and e); c) and d); c) and e); or d) and e). The PKR1 agonist may meet three of these features, for instance a), b) and c); a), b) and d); a), b) and e); a), c) and d); a), c) and e); a), d) and e); b), c) and d); b), c) and e); b), d) and e); or c), d) and e). The PKR1 agonist may meet four of these features, for instance a), b), c) and d); a), b), c) and e); a), b), d) and e); b), c), d) and e); or a), c), d) and e). The PKR1 agonist may also meet all of these features, i.e. a), b), c), d) and e).

The PKR1 agonist may be selected from the group consisting of

| Name of agonist | Formula |
|---|---|
| IS1 | 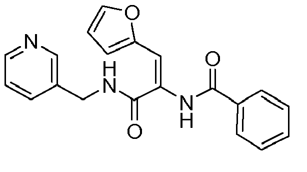 |
| IS2 | 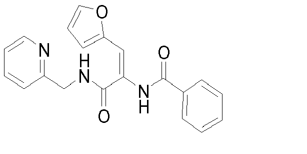 |
| IS3 | 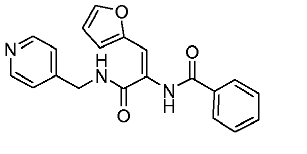 |
| IS4 | 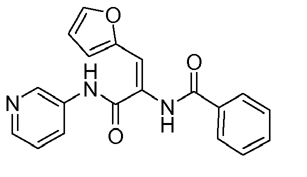 |
| IS5 | 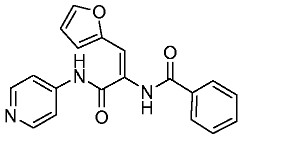 |
| IS6 | 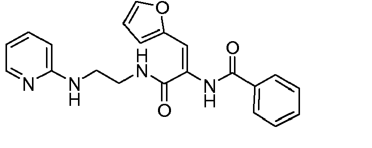 |
| IS7 | 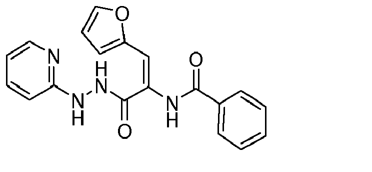 |
| IS8 | 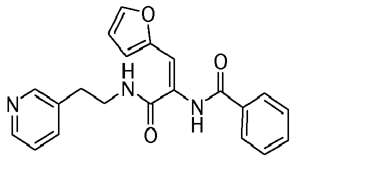 |
| IS9 | 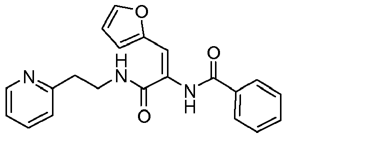 |
| IS10 | 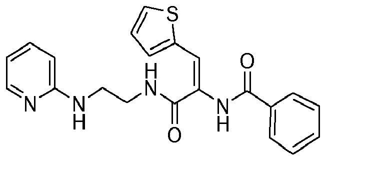 |
| IS11 | 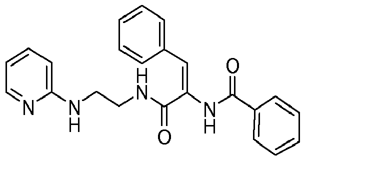 |
| IS12 | 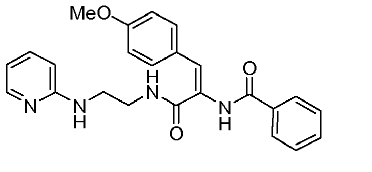 |
| IS14 | 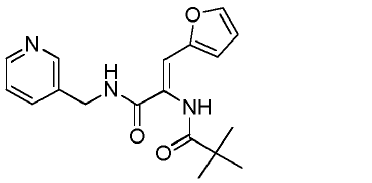 |
| IS15 | 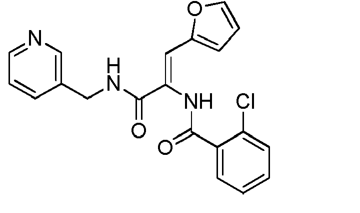 |
| IS16 | 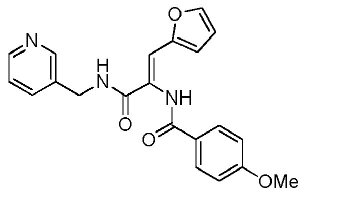 |
| IS17 | 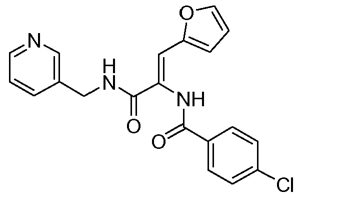 |
| IS18 | 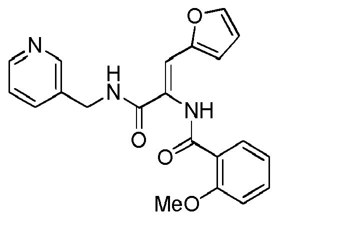 |
| IS19 | 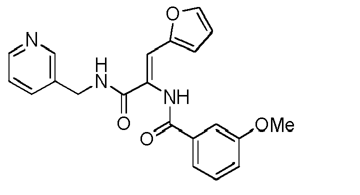 |

In a particular embodiment, the PKR1 agonist has the formula (I), (II), (III) or (IV), m, n, R¹, R² and R³ are defined as in formula (I) and in the above disclosed embodiments, with the proviso that when m is 0, n is 0, R¹ is a hydrogen atom, R² is a furan group, R³ is a phenyl group then the agonist has the formula (II) or (IV).

The present invention also concerns a PKR1 agonist of formula (I), (II), (III) or (IV), wherein m, n, R¹, R² and R³ have the same meaning as in the above disclosed embodiments, with the proviso that when m is 0, n is 1, R¹ is a hydrogen atom and R² is a furan group, then R³ is not a phenyl group or a phenyl group substituted in para by a methoxy group; and when m is 0, n is 0, R¹ is a hydrogen atom and R² is a furan group, then R³ is not a phenyl group.

The present invention further concerns a PKR1 agonist of formula (I), (II), (III) or (IV), wherein n, R¹, R² and R³ have the same meaning as in the above disclosed embodiments, and m is 1. In an embodiment, m is 1 and n is 2. In a particular embodiment, m is 1; n is selected from 0 and 2, R¹ is a hydrogen atom; R² is selected from the group consisting of 5-membered-ring O- or S-heterocycle and a phenyl group, optionally substituted by a (C₁-C₃)-alkoxy group, preferably a methoxy group; and R³ is a phenyl group. Preferably, R² is selected from the group consisting of a furan, a thiophene, a phenyl and a methoxyphenyl group. More preferably, R² is selected from the group consisting of a furan and a methoxyphenyl group.

The present invention further concerns a PKR1 agonist of formula (I), (II), (III) or (IV), wherein m, R¹, R² and R³ have the same meaning as in the above disclosed embodiments, and n is 2, with the proviso that when m is 0, R¹ is a hydrogen atom, R² is a furan group and R³ is a phenyl group then the agonist has the formula (III) or (IV). In an embodiment, R¹ is a hydrogen atom; R² is selected from the group consisting of a 5-membered-ring O- or S-heterocycle and a phenyl group, optionally substituted by a (C₁-C₃)-alkoxy group, preferably a methoxy group; and R³ is a phenyl group. Preferably, R² is selected from the group consisting of a furan, a thiophene, a phenyl and a methoxyphenyl group. More preferably, R² is selected from the group consisting of a furan and a methoxyphenyl group.

The present invention further concerns a PKR1 agonist of formula (I), (II), (III) or (IV), wherein m, n, R¹ and R² have the same meaning as in the above disclosed embodiments, and R³ is a (C₁-C₆)-alkyl group, optionally substituted by at least one substituent selected from a hydroxyl, a carboxylic group, an aldehyde, a halogen atom and a thiol. In an embodiment, R³ is a (C₁-C₆)-alkyl group. In a particular embodiment, R³ is a tert-butyl group. In a more particular embodiment, R¹ is a hydrogen atom; R² is selected from the group consisting of a 5-membered-ring O- or S-heterocycle, preferably a furan group, and a phenyl group, optionally substituted by a (C₁-C₃)-alkoxy group, preferably a methoxy group; and R³ is a (C₁-C₆)-alkyl group, optionally substituted by at least one substituent selected from a hydroxyl, a carboxylic group, an aldehyde, a halogen atom and a thiol. Preferably, R³ is a (C₁-C₆)-alkyl group. More preferably, R³ is a tert-butyl group.

The present invention further concerns a PKR1 agonist of formula (I), (II), (III) or (IV), wherein m, n, R¹ and R² have the same meaning as in the above disclosed embodiments, and R³ is selected from the group consisting of a phenyl group and a 5-membered-ring heterocycle, substituted by at least one substituent selected from the group consisting of a (C₁-C₃)-alkyl, a (C₁-C₃)-alkoxy group and a halogen atom, with the proviso that when m is 0, n is 1, R¹ is a hydrogen atom, R² is a furan group and R³ is a phenyl group substituted by a methoxy, then the methoxy group is in meta- or ortho-position. In an embodiment, R³ is selected from the group consisting of a phenyl group and a phenyl group substituted by at least one substituent selected from the group consisting of a (C₁-C₃)-alkyl, a (C₁-C₃)-alkoxy group and a halogen atom, preferably from the group consisting of methyl, methoxy and chlorine.

The present invention further concerns a PKR1 agonist of formula (I), (II), (III) or (IV), wherein m, n, R¹ and R³ have the same meaning as in the above disclosed embodiments, and R² is selected from the group consisting of a 5 to 7-membered-ring S-heterocycle and a phenyl group, optionally substituted by at least one substituent selected from the group consisting of a (C₁-C₃)-alkyl, a (C₁-C₃)-alkoxy group or a halogen atom. In an embodiment, R² is selected from the group consisting of a 5-membered-ring S-heterocycle, preferably a thiophene group, a phenyl and a methoxyphenyl group. In a particular embodiment, m is 1; n is 2; R¹ is a hydrogen atom; R² is selected from the group consisting of a 5-membered-ring S-heterocycle, preferably a thiophene group, a phenyl and a methoxyphenyl group; and R³ is a phenyl group.

The present invention further concerns a PKR1 agonist as described above as a medicament. The PKR1 agonist can be a PKR1 agonist according to any of the above embodiments and can optionally be selected from the group consisting of IS1, IS2, IS3, IS4, IS5, IS6, IS7, IS8, IS9, IS10, IS11, IS12, IS14, IS15, IS16, IS17, IS18 and IS19, preferably from the group consisting of IS6, IS7, IS8, IS10, IS11, IS12, IS14, IS15, IS17, IS18 and IS19, more preferably from the group consisting of IS3, IS5, IS6 and IS12.

The present invention also concerns a PKR1 agonist as described above as a cardioprotective agent. The PKR1 agonist can be a PKR1 agonist according to any of the above embodiments and can optionally be selected from the group consisting of IS1, IS2, IS3, IS4, IS5, IS6, IS7, IS8, IS9, IS10, IS11, IS 12, IS 14, IS15, IS16, IS17, IS18 and IS19, preferably from the group consisting of IS6, IS7, IS8, IS10, IS11, IS12, IS14, IS15, IS17, IS18 and IS19, more preferably from the group consisting of IS3, IS5, IS6 and IS12.

The present invention also concerns a PKR1 agonist as described above as a neuroprotective agent. The PKR1 agonist can be a PKR1 agonist according to any of the above embodiments and can optionally be selected from the group consisting of IS1, IS2, IS3, IS4, IS5, IS6, IS7, IS8, IS9, IS10, IS11, IS 12, IS 14, IS15, IS16, IS17, IS18 and IS19, preferably from the group consisting of IS6, IS7, IS8, IS10, IS11, IS12, IS14, IS15, IS17, IS18 and IS19, more preferably from the group consisting of IS3, IS5, IS6 and IS12.

The PKR1 agonist activity of the compounds according to the invention may be assessed by any method known by the skilled person. For example, the PKR1 agonist activity may be assessed by any of the methods disclosed in the experimental section: the measure of the capacity to induce in vitro angiogenesis, the measure of the capacity to activate the PKR1 signalling pathway (e.g. by measurement of the intracellular calcium), or the capacity to induce to internalization of the receptor.

The PKR1 agonists of the invention can be synthesized by any process known by the man skilled in the art. Such processes are described, for example, in the articles of Erlenmeyer and Plöchl (Erlenmeyer, 1893; Plöchl, 1884). As illustrative example, the synthesis of the agonists IS1, IS2, IS3, IS4, IS5, IS6, IS7, IS8, IS9, IS10, IS11, IS12, IS14, IS15, IS16, IS17, IS18 and IS19, is detailed in the present application in the experimental section.

### Pharmaceutical composition

The present invention also concerns a pharmaceutical composition comprising a PKR1 agonist as described above and a pharmaceutically acceptable carrier and/or excipient.

The pharmaceutical composition of the invention may comprise one or several PKR1 agonists according to any of the above embodiments. For example, the composition may comprise 1, 2, 3, 4, 5, 6, 7, 8, 9 or 10 PKR1 agonists as described above.

In a particular embodiment, the pharmaceutical composition comprises one or several PKR1 agonist selected from the group consisting of IS1, IS2, IS3, IS4, IS5, IS6, IS7, IS8, IS9, IS11, IS12, IS14, IS15, IS16, IS17, IS18 and IS19. Preferably, the pharmaceutical composition comprises one or several PKR1 agonist selected from the group consisting of IS1, IS3, IS5, IS6, IS11, IS12, IS14, IS15, IS17 and IS18. More preferably, the pharmaceutical composition comprises one or several PKR1 agonist selected from the group consisting of IS6, IS11, IS12, IS14, IS15, IS17 and IS18.

In another embodiment, the pharmaceutical composition comprises one or several PKR1 agonist selected from the group consisting of IS6, IS7, IS8, IS11, IS12, IS14, IS15, IS17, IS18 and IS19.

In a preferred embodiment, the pharmaceutical composition of the invention comprises a) IS5, b) IS6, c) IS3, d) IS5 and IS6, e) IS6 and IS3, f) IS5 and IS3 or g) IS 6, IS3 and IS5.

The pharmaceutical composition of the invention is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art. Possible pharmaceutical compositions include those suitable for oral, rectal, topical (including transdermal, buccal and sublingual), or parenteral (including subcutaneous, intramuscular, intravenous and intradermal) administration. For these formulations, conventional excipient can be used according to techniques well known by those skilled in the art. The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient. For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. For transdermal administration, the composition can be formulated into ointment, cream or gel form and appropriate penetrants or detergents could be used to facilitate permeation, such as dimethyl sulfoxide, dimethyl acetamide and dimethylformamide. For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. In a preferred embodiment, the pharmaceutical composition of the invention is suitable for parenteral or oral administration.

Pharmaceutical composition according to the invention may be formulated to release the active drug substantially immediately upon administration or at any predetermined time or time period after administration.

In a particular embodiment, the pharmaceutical composition according to the invention comprises 10 mg to 2 g of PKR1 agonist of the invention. Preferably, pharmaceutical composition according to the invention comprises 100 mg to 800 mg of PKR1 agonist of the invention.

The pharmaceutical composition according to the invention can further comprise one or more other active compounds associated with pharmaceutically acceptable excipients and/or carriers. For example, the agonists of the invention can be associated with other PKR agonists such as prokineticin-2 or the prokineticin 2β peptide as described in the international patent application WO 2005/097826. They also can be associated with other drugs such as drugs used for the treatment of vascular diseases, neurodegenerative diseases, diseases involving impaired gastrointestinal motility, obesity, Kallmann syndrome, normosmic hypogonadotropic hypogonadism, disturbances of circadian rhythm or cancers.

The agonists of the invention can be associated with drugs used for the treatment of vascular diseases. Examples of such drugs include, but are not limited to, ACE inhibitors (e.g., enalapril, captopril, lisinopril and ramipril), beta-blockers (e.g. alprenolol, bucindolol, carteolol, carvedilol, labetalol, nadolol, penbutolol, pindolol, propranolol, acebutolol, atenolol, betaxolol, bisoprolol, celiprolol and esmolol), aldosterone antagonists (e.g. spironolactone, eplerenone, canrenone, prorenone and mexrenone) or vasodilators, or a combination thereof.

The agonists of the invention can also be associated with drugs used for the treatment of neurodegenerative diseases. Examples of such drugs include, but are not limited to, riluzole, acetylcholinesterase inhibitors (e.g. donepezil, galantamine and rivastigmine), memantine, Levodopa (or L-DOPA), dopamine agonists (e.g. bromocriptine, pergolide, pramipexole, ropinirole , piribedil, cabergoline, apomorphine, and lisuride), MAO-B inhibitors (e.g. selegiline and rasagiline), tetrabenazine, amantadine or valproic acid, or a combination thereof.

The agonists of the invention can be associated with drugs used for the treatment of diseases involving impaired gastrointestinal motility, obesity. Examples of such drugs include, but are not limited to, mesalazine, immunosuppressants (e.g. prednisone, TNF inhibition, azathioprine, methotrexate and 6-mercaptopurine), proton pump inhibitors (e.g. omeprazole, esomeprazole, pantoprazole, lansoprazole and rabeprazole), mosapride or laxatives, or a combination thereof.

The agonists of the invention can be associated with one or several hormones used to treat Kallman syndrome and normosmic hypogonadotropic hypogonadism such as human chorionic gonadotropin (hCG), testosterone, estrogen or progestins, or a combination thereof.

The agonists of the invention can also be associated with orlistat used to treat obesity.

The other active compound associated with the PKR1 agonist of the invention may also be a drug having cardiotoxic and/or neurotoxic side effects.

In a particular embodiment, the pharmaceutical composition according to the invention comprises at least one PKR1 agonist of the invention and an antiviral agent, in particular an antiviral agent having cardiotoxic and/or neurotoxic side effects. The antiviral agent may be selected from the group consisting of D4T (2'-3'-didehydro-2'-3'-dideoxythymidine or Stavudine), ddI (2'3'-dideoxyinosine or Didanosine) and ddC (2'-3'-dideoxycytidine or Zalcitabine).

In another particular embodiment, the pharmaceutical composition according to the invention comprises at least one PKR1 agonist of the invention and an antineoplastic agent, in particular an antineoplastic agent having cardiotoxic and/or neurotoxic side effects. The antineoplastic agent may be selected from the group consisting of antineoplastic agents belonging to platinum, taxane, anthracycline and vinca alkaloid families. In particular, the antineoplastic agent may be selected from the group consisting of cisplatin, carboplatin, oxaplatin, picoplatin, tetraplatin, satraplatin, paclitaxel, docetaxel, doxorubicin, daunorubicin, idarubicin, detorubicin, carminomycin, epirubicin, morpholinodoxorubicin, morpholinodaunorubicin, methoxymorpholinyldoxorubicin, vinblastine, vincristine, vindesine, vinorelbine, cyclophosphamide, fluorouracil, rituximab, arsenic trioxide, trastuzumab, thalidomide, etoposide, pentastatin, cytarabine, interferons, busulfan and derivatives and combinations thereof.

The PKR1 agonists of the invention can also be associated with other neuroprotective and/or cardioprotective agents.

The present invention also concerns a product containing a PKR1 agonist of the invention and an antineoplastic agent, as a combined preparation for simultaneous, separate or sequential use in the treatment of cancer.

### Uses of PKR1 agonists and pharmaceutical compositions comprising thereof

Previous studies have demonstrated that :
- Prokineticin-2 prevents the apoptotic death of cardiomyocytes induced by oxidative stress and promotes cardiomyocyte survival and angiogenesis (Urayama et al., 2007);
- Prokineticins have a role in appetite regulation. In particular, prokineticin 2 has anorectic effect and can thus potently inhibit food intake (Gardiner et al., 2010);
- Prokineticins potently and specifically stimulate the contraction of gastrointestinal smooth muscle through the activation of the prokineticin receptor (Li et al., 2001);
- Prokineticin-2 supports neuronal survival and protects neurons against excitotoxic death, and is involved in postnatal and adult olfactory bulb neurogenesis (Melchiorri et al., 2001; Ng et al., 2005);
- The administration of prokineticin-2 in adult rat brain results in increased neurogenesis (patent US 7,323,334);
- Prokineticins and their receptors are associated with Kallmann syndrome and normosmic hypogonadotropic hypogonadism (Hardelin and Dodé, 2008; Abreu et al., 2010);
- Prokineticins and their receptors are associated with disturbances of circadian rhythm (Cheng et al., 2002).

Consequently, the present invention concerns a pharmaceutical composition or a PKR1 agonist as described above for use for the prevention or treatment of a disease or a disorder selected from the group consisting of a vascular disease, a neurodegenerative disease, a disease involving impaired gastrointestinal motility, obesity, Kallmann syndrome, normosmic hypogonadotropic hypogonadism, hyperthyroidism, in particular Familial isolated hyperparathyroidism (FIHP), disturbances of circadian rhythm, sleeping disorders and a pregnancy and placental function-related disorder. Preferably, the disease or disorder is selected from the group consisting of a vascular disease, a neurodegenerative disease, a disease involving impaired gastrointestinal motility, obesity, Kallmann syndrome, normosmic hypogonadotropic hypogonadism, and disturbances of circadian rhythm.

The cardiovascular disease may be selected from the group consisting of myocardial infarction, acute coronary syndrome, ischemic stroke, abdominal aorta aneurysm, cerebral aneurysm, ischemic heart disease, coronary heart disease, peripheral arterial disease, restenosis, angina pectoris, diabetic angiopathy and diabetes-mediated cardiovascular complications.

The neurodegenerative disease may be selected from the group consisting of Parkinson's disease and other parkinsonian disorders, Alzheimer's disease and other dementing neurodegenerative disorders, amyotrophic lateral sclerosis, multiple sclerosis, Creutzfeldt-Jakob disease, Huntington's disease and neuronal degeneration associated to multiple sclerosis.

The disease involving impaired gastrointestinal motility may be selected from the group consisting of irritable bowel syndrome, diabetic gastroparesis, postoperational ileus, chronic constipation, and gastroesophageal reflux disease, Hirchsprung's disease (congenital colonic aganglionosis) and chronic dyspepsia.

The pregnancy and placental function-related disorder may be selected from the group consisting of placental dysfunction, preeclampsia, fetal inflammatory response syndrome and antiphospholipid syndrome.

The present invention also concerns a pharmaceutical composition or a PKR1 agonist as described above for use for the prevention or treatment of a disease associated with insufficient prokineticin receptor activity.

The present invention also concerns the use of a PKR1 agonist of the invention for preparing a medicament for preventing or treating a disease or a disorder selected from the group consisting of a vascular disease, a neurodegenerative disease, a disease involving impaired gastrointestinal motility, obesity, Kallmann syndrome, normosmic hypogonadotropic hypogonadism, hyperthyroidism, in particular Familial isolated hyperparathyroidism (FIHP), disturbances of circadian rhythm, sleeping disorders and a pregnancy and placental function-related disorder.

In another aspect, the present invention further concerns a method for preventing or treating a disease or a disorder in a subject, wherein said disease or disorder is selected from the group consisting of a vascular disease, a neurodegenerative disease, a disease involving impaired gastrointestinal motility, obesity, Kallmann syndrome, normosmic hypogonadotropic hypogonadism, hyperthyroidism, in particular Familial isolated hyperparathyroidism (FIHP), disturbances of circadian rhythm, sleeping disorders and a pregnancy and placental function-related disorder, and comprising administering a therapeutically active amount of a PKR1 agonist according to the invention to said subject. By a "therapeutically effective amount" is intended an amount of PKR1 agonist of the invention administered to a patient that is sufficient to provide a therapeutic effect. It is to be understood that the total amount of the PKR1 agonist of the invention may vary depending on the volume of blood plasma of the patient. Suitable means and measures to determine the therapeutically effective amount are available to the person skilled in the art. In a particular embodiment, the method of the invention for preventing or treating a disease or a disorder in a subject, wherein said disease or disorder is selected from the group consisting of a vascular disease, a neurodegenerative disease, a disease involving impaired gastrointestinal motility, obesity, Kallmann syndrome, normosmic hypogonadotropic hypogonadism, hyperthyroidism, in particular Familial isolated hyperparathyroidism (FIHP), disturbances of circadian rhythm, sleeping disorders and a pregnancy and placental function-related disorder, comprises administering 0.2 to 30 mg / kg of body weight / day of a PKR1 agonist of the invention to said subject. All embodiments as disclosed above for a pharmaceutical composition or a PKR1 agonist of the invention for use for the prevention or treatment of a disease or a disorder selected from the group consisting of a vascular disease, a neurodegenerative disease, a disease involving impaired gastrointestinal motility, obesity, Kallmann syndrome, normosmic hypogonadotropic hypogonadism, hyperthyroidism, in particular Familial isolated hyperparathyroidism (FIHP), disturbances of circadian rhythm, sleeping disorders and a pregnancy and placental function-related disorder, are also encompassed in this aspect.

Due to their cardioprotective and neuroprotective activities, the PKR1 agonists of the invention and the pharmaceutical compositions comprising thereof, may be used to prevent or to limit the neurotoxicity and/or cardiotoxicity of a compound, in particular of an antiviral or antineoplastic agent. The antiviral agent may be selected from the group consisting of D4T (2'-3'-didehydro-2'-3'-dideoxythymidine or Stavudine), ddI (2'3'-dideoxyinosine or Didanosine) and ddC (2'-3'-dideoxycytidine or Zalcitabine). The antineoplastic agent may be selected from the group consisting of antineoplastic agents belonging to platinum, taxane, anthracycline and vinca alkaloid families. In particular, the antineoplastic agent may be selected from the group consisting of cisplatin, carboplatin, oxaplatin, picoplatin, tetraplatin, satraplatin, paclitaxel, docetaxel, doxorubicin, daunorubicin, idarubicin, detorubicin, carminomycin, epirubicin, morpholinodoxorubicin, morpholinodaunorubicin, methoxymorpholinyldoxorubicin, vinblastine, vincristine, vindesine, vinorelbine, cyclophosphamide, fluorouracil, rituximab, arsenic trioxide, trastuzumab, thalidomide, etoposide, pentastatin, cytarabine, interferons, busulfan and derivatives and combinations thereof.

The present invention also concerns the use of a PKR1 agonist of the invention for preparing a medicament for preventing or limiting the cardiotoxicity and/or neurotoxicity of a compound, in particular the cardiotoxicity and/or neurotoxicity of an antiviral or antineoplastic agent.

In another aspect, the present invention further concerns a method for preventing or limiting the cardiotoxicity and/or neurotoxicity of a compound in a subject, in particular the cardiotoxicity and/or neurotoxicity of an antiviral or antineoplastic agent, comprising administering a therapeutically active amount of a PKR1 agonist according to the invention. In this aspect, by a "therapeutically effective amount" is intended an amount of PKR1 agonist of the invention administered to a patient that is sufficient to prevent the cardiomyocyte and/or neuronal cell death. It is to be understood that the total amount of the PKR1 agonist of the invention may vary depending on the volume of blood plasma of the patient. Suitable means and measures to determine the therapeutically effective amount are available to the person skilled in the art. In a particular embodiment, the method of the invention for preventing or limiting the cardiotoxicity and/or neurotoxicity of a compound in a subject comprises administering 0.2 to 30 mg / kg of body weight / day of a PKR1 agonist of the invention to said subject.

The following examples are given for purposes of illustration and not by way of limitation.

### Examples

### Synthesis and NMR characterization of PKR1 agonists

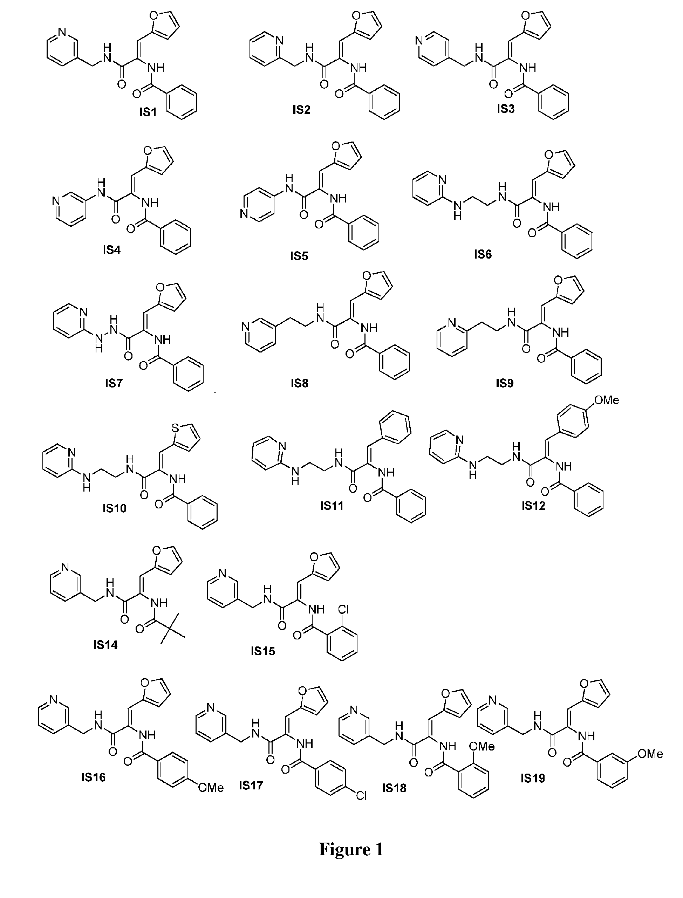

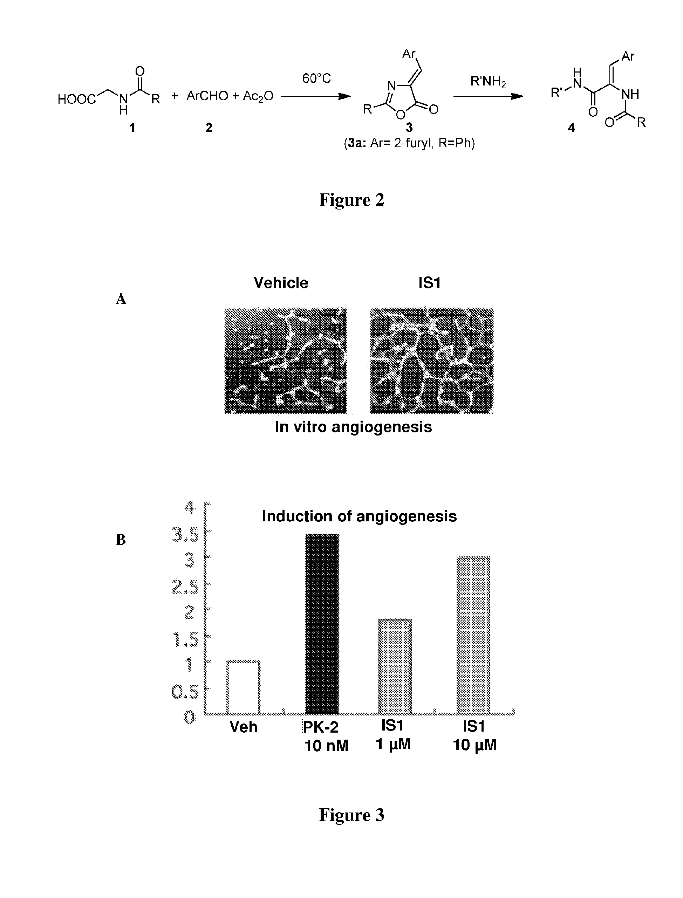

IS compounds (Figure 1) were synthesized according to a general method originally developed by Erlenmeyer and Plöchl (Erlenmeyer, 1893; Plöchl, 1884). Condensation of acylated glycine 1 with an aldehyde 2 in the presence of acetic anhydride afforded the azlactone 3, which was condensed to an amine to afford 4 (Figure 2).

### IS1 to IS3, IS6, IS8 to IS12 and IS14 to IS19

An amine (4 mmol) and an azlactone 3 (4 mmol) in suspension in CH₂Cl₂/EtOH (8 mL, 1/1) was sonicated for 10 min. After stirring overnight at room temperature, the solvent was removed under vacuum, and the residue was purified by flash chromatography (EtOAc to EtOAc/EtOH, 80/20) to give the expected product a white solid (purity > 97%).

| | |
|---|---|
| 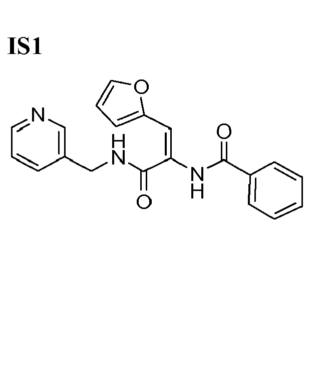 | **¹H NMR** (400 MHz, DMSO-d₆): 9.77 (s, 1H), 8.68 (m, 1H), 8.5 8 (s, 1H), 8.47 (d, *J* = 4.6 Hz, 1H), 8.13 (m, 2H), 7.77 (d, *J* = 7.8 Hz, 1H), 7.62 (m, 2H), 7.55 (m, 2H), 7.33 (m, 2H), 6.69 (d, *J* = 3.4 Hz, 1H), 6.52 (m, 1H), 4.49 (d, *J* = 6.0 Hz, 2H); **RMN ¹³C** (100 MHz, DMSO): 40.3, 111.7, 113.5, 117.7, 122.8, 126.8, 127.7, 127.8, 131.2, 133.6, 134.7, 143.6, 147.5, 148.5, 149.6, 164.4, 165.7 ppm. LC-MS (ESI): calculated: 347.1; found: 348.0 (M+H⁺)⁺. |
| 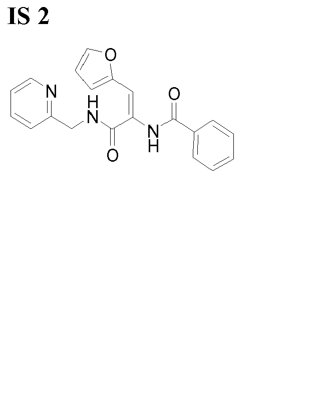 | **¹H NMR** (400 MHz, CDCl₃): 8.57 (s, 1H), 8.42 (d, *J* = 4.9 Hz, 1H), 7.92 (d, *J* = 7.5 Hz, 2H), 7.61 (m, 2H), 7.53 (d, *J* = 7.8Hz, 1H), 7.43 (m, 3H), 7.32 (d, *J* = 7.8 Hz, 1H), 7.11 (m, 1H), 6.94 (s, 1H), 6.49 (d, *J* = 3.5, 1H), 6.41 (dd, *J* = 1.9, 3 .6 Hz, 1H), 4.33 ( d, *J* = 5.3 Hz, 2H); **¹³C NMR** (100 MHz, CDCl₃):166.5, 165.1, 156.8, 150.5, 149.0, 144.1, 137.0, 133.6, 132.4, 128.9, 127.8, 127.4, 122.4, 122.2, 114.6, 114.0, 112.3, 45.1. LC-MS (ESI): calculated: 347.1; found 348.2 (M+H⁺)⁺. |
| 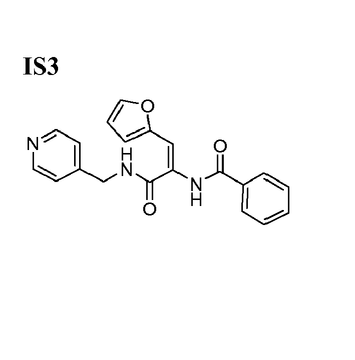 | **1H NMR** (300 MHz, CDCl₃): 8.59 (s, 1H), 8.49 (m, 2H), 7.93 (m, 2H), 7.56 (m, 1H), 7.47 (m, 3H), 7.25 (s, 1H), 7.12 (m, 1H), 6.94 (s, 1H), 6.52 (d, *J* = 3.4 Hz, 1H), 6.44 (m, 1H), 4.53 (d, *J*= 6.2 Hz, 2H); **¹³C NMR** (100 MHz, DMSO-d₆): 165.9, 164.7, 149.5, 148.9, 148.1, 143.5, 133.3, 131.2, 127.8, 127.5, 126.4, 121.7, 117.3, 113.6, 111.6, 41.7. LC-MS (ESI): calculated 347.1; found: 348.2 (M+H)⁺. |
| 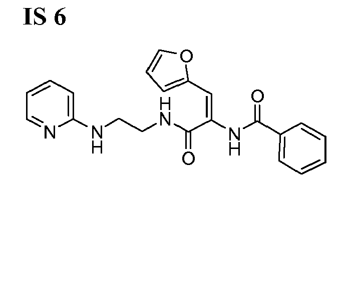 | **¹H NMR** (400 MHz, CDCl₃): 8.75 (s, 1H), 7.87 (m, 3H), 7.58 (m, 1H), 7.48 (t, *J* = 7.4 Hz, 1H), 7.38 (m, 3H), 7.24 (m, 1H), 6.69 (s, 1H), 6.38 (m, 4H), 5.29 (m, 1H), 3.46 (m, 4H); **¹³C NMR** (100 MHz, CDCl₃): 166.4, 165.8, 159.0, 150.4, 147.6, 143.9, 137.3, 133.3, 132.4, 128.8, 127.8, 127.6, 114.2, 113.3, 112.8, 112.2, 108.6, 41.1, 41.0. LC-MS (ESI): calculated: 376.2; found: 377.2 (M+H⁺)⁺. |
| 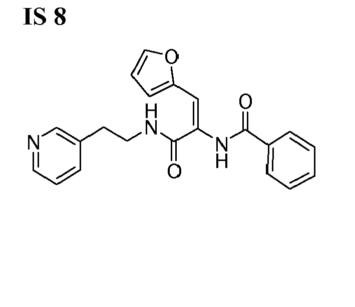 | **¹H NMR** (400 MHz, CDCl₃): 8.46 (s, 2H), 8.41 (m, 1H), 7.91 (m, 2H), 7.51 (m, 5H), 7.15 (m, 1H), 6.83 (s, 1H), 6.48 (m, 3H), 3.60 (q, *J* = 6.7 Hz, 2H), 2.91 (q, *J* = 7 Hz, 2H); **¹³ C NMR** (100 MHz, CDCl₃): 166.5, 165.2, 150.4, 150.3, 148.0, 144.1, 136.7, 134.8, 133.3, 132.6, 129.0, 127.8, 127.7, 123.7, 114.6, 113.6, 112.3, 41.1, 33.0. LC-MS (ESI): calculated: 361.1; found: 362.2 (M+H⁺)⁺. |
| 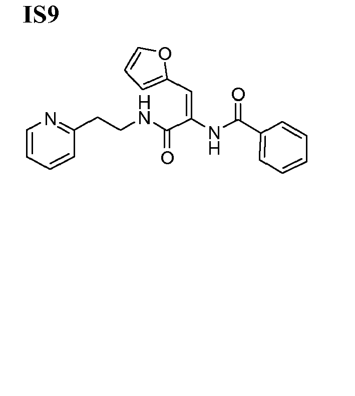 | **RMN ¹H** (400 MHz, CDCl₃): 8.041 (s, 1H), 8.18 (d, *J* = 4.3 Hz, 1H), 7.90 (d, *J* = 7.4 Hz, 2H), 7.51 (m, 5H), 7.40 (d, *J* = 1.8 Hz, 1H), 7.14 (d, *J* = 7.8 Hz, 1H), 6.99 (ddd, *J*=0.9, 5.0, 7.5 Hz, 1H), 6.92 (s, 1H), 6.48 (d, *J* = 3.4 Hz, 1 H), 6.41 (dd, *J* = 1. 8, 3.5 Hz, 1H), 3.73 (dd, *J* = 5.9, 12.4 Hz, 2H), 2.99 (t, *J* = 6.3Hz, 2H); **¹³C NMR** (100 MHz, CDCl₃): 166.3, 164.7, 160.0, 150.6, 149.1, 144.0, 136.8, 133.7, 132.4, 128.9, 127.8, 127.6, 123.8, 121.6, 114.4, 114.0, 112.3, 39.4, 36.8. LC-MS (ESI): calculated: 361.1; found: 362.2 (M+H⁺)⁺. |
| **IS10** | **¹H NMR** (400 MHz, CDCl₃): 8.41 (s, 1H), 7.93 (d, *J* = 7.7Hz, 2H), 7.82 (d, *J* = 4.6Hz, 1H), 7.73 (s, 1H), 7.47 (m, 2H), 7.32 (m, 4H), 7.15 (d, *J* = 3.6 Hz, 1H), 6.99 (m, 1H), |
| 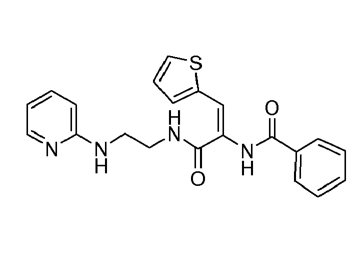 | 6.44 (m, 1H), 6.32 (d, *J* = 8.4 Hz, 1H), 5.16 (s, 1 H), 3.38 (s, 4H); **¹³ C NMR** (100 MHz, CDCl₃): 167.2, 165.9, 159.0, 147.6, 137.6, 136.8, 133.2, 132.4, 132.2, 129.5, 128.8, 128.0, 127.4, 126.2, 125.1, 113.0, 108.3, 41.4, 41.3. LC-MS (ESI): calculated: 392.1; found: 393.2 (M+H⁺)⁺. |
| 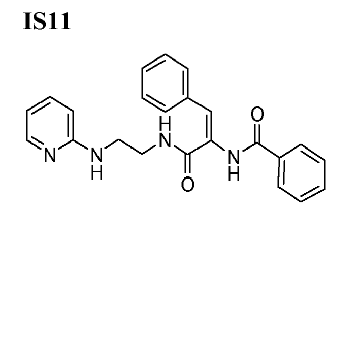 | **¹H NMR** (400 MHz, CDCl₃): 8.10 (s, 1H), 7.89 (m, 1H), 7.81 (d, *J* = 7.5 Hz,2H), 7.57 (s, 1H), 7.50 (m, 1H), 7.33 (m, 8H), 6.93 (s, 1H), 6.47 (m, 1H), 6.40 (d, *J* = 8.4Hz, 1H), 5.06 (t, *J* = 5.4 Hz, 1H), 3.53 (m, 4H); **¹³C NMR** (100 MHz, CDCl₃): 167.0, 166.7, 158.9, 147.6, 137.4, 134.1, 133.0, 132.2, 129.9, 129.4, 128.9, 128.8, 128.7, 127.9, 127.5, 112.9, 108.4, 41.2. LC-MS (ESI): calculated: 386.2; found: 387.2 (M+H⁺)⁺. |
| 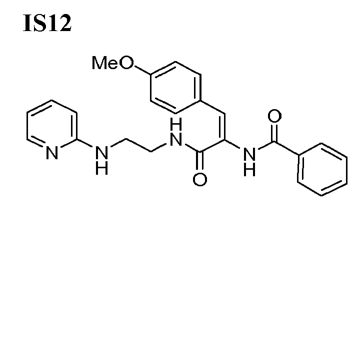 | **¹H NMR** (400 MHz, CDCl₃): 8.20 (s, 1H), 7.88 (m, 1H), 7.83 (d, *J* = 7.4 Hz, 2H), 7.56 (s, 1 H), 7.49 (m, 1H), 7.34 (m, 5 H), 6.93 (s, 1H), 6.79 (d, *J* = 8.8 Hz, 2H), 6.46 (m, 1 H), 6.3 (d, *J* = 8.4 Hz, 1H), 5.11 (m, 1H), 3.48 (m, 4H); **¹³ C NMR** (100 MHz, CDCl₃): 167.3, 166.8, 160.0, 158.9, 147.6, 137.4, 133.0, 132.1, 131.2, 128.6, 128.1, 127.9, 127.7, 126.5, 114.1, 112.8, 108.4, 55.4, 41.2, 41.0. LC-MS (ESI): calculated: 416.2; found: 417.2 (M+H⁺)⁺. |
| 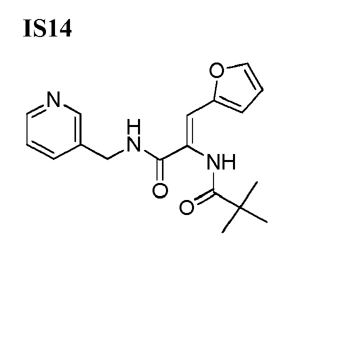 | **¹H NMR** (400 MHz, CDCl₃): 8.51 (d, *J* = 1.6 Hz, 1H), 8.46 (dd, *J* = 1.1, 4.6 Hz, 1H), 8.05 (s, 1H), 7.70 (d, *J* = 7.8 Hz, 1H), 7.45 (s, 1H), 7.23 (dd, *J* = 4.8, 7.8 Hz, 1H), 6.90 (t, *J* = 5.3Hz, 1 H), 6.73 (s, 1H), 6.46 (m, 2H), 4.52 (d, *J* = 5.9 Hz, 2H), 1.29 (s, 9H). **¹³C NMR** (100 MHz, CDCl₃): 178.2, 165.5, 150.5, 149.2, 148.9, 143.8, 135.8, 134.2, 127.9, 123.8, 114.5, 112.8, 112.4, 41.6, 39.6, 27.6. LC-MS (ESI): calculated: 327.2; found: 328.2 (M+H⁺)⁺. |
| **IS 15** | **¹H NMR** (400 MHz, CDCl₃): 8.65 (s, 1H), 8.41 (s, 1H), 8.35 (dd, *J* = 1.5, 4.8 Hz, 1H), 7.68 (d, *J* = 7.7 Hz, 2H), 7.41 (s, 1H), 7.36 (d, *J* = 3.8 Hz, 2H), 7.31 (m, 1H), 7.17 (dd, *J* = 4.8, 7.8 Hz, 1H), 7.12 (t, J = 5.9 Hz, 1H), 7.06 (s, 1H), 6.56 |
| 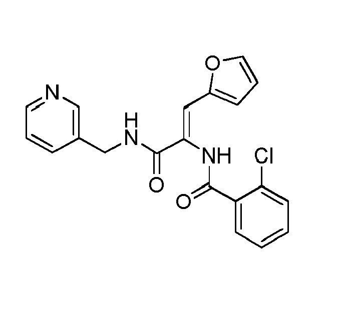 | (d, *J* = 3.4 Hz, 1H), 6.42 (dd, *J* = 1.9,3.4 Hz, 1H), 4.49 (d, *J* = 5.8 Hz, 2H); **¹³C NMR** (100 MHz, CDCl₃): 166.0, 164.8, 150.0, 149.1, 148.7, 144.5, 135.9, 134.5, 134.0, 132.0, 131.0, 130.4, 130.1, 127.5, 125.5, 123.8, 120.2, 116.6, 115.6, 112.4, 41.6. LC-MS (ESI): calculated: 381.1; found: 382.0 (M+H⁺)⁺. |
| 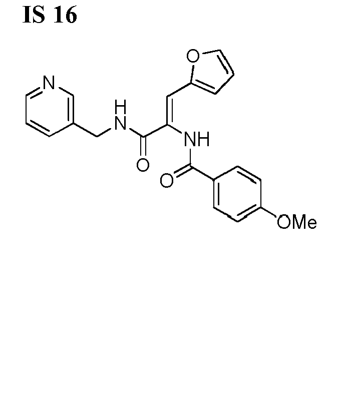 | **¹H NMR** (400 MHz, DMSO-d₆): 9.72 (s, 1H), 8.71 (t, *J* = 5.8 Hz, 1H), 8.53 (s, 1H), 8.43 (d, *J* = 4.1 Hz, 1H), 8.05 ( d, *J* = 8.5 Hz, 2H), 7.72 (m, 2H), 7.33 (dd, *J* = 4.8, 7.5 Hz, 1H), 7.19 (s, 1H), 7.06 (d, *J* = 8.7 Hz, 2H), 6.68 (d, *J* = 3.3 Hz, 1H), 6.56 (s, 1H), 4.40 (d, *J*=5.8 Hz, 2H), 3.84 (s, 3H). **¹³C NMR** (100 MHz, DMSO-d₆): 165.4, 164.7, 162.0, 149.8, 148.6, 147.8, 144.4, 135.2, 134.9, 129.9, 127.5, 126.1, 123.2, 117.6, 113.7, 113.5, 112.2, 55.4, 40.4. LC-MS (ESI): calculated: 377.1; found: 378.2 (M+H)⁺. |
| 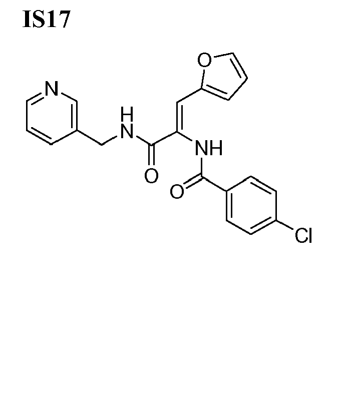 | **¹H NMR** (400 MHz, DMSO-d₆): 9.94 (s, 1H), 8.75 (t, *J* = 6.0 Hz, 1H), 8.52 (d, *J*=1.8 Hz, 1H), 8.43 (dd, *J* = 1.4, 4.6 Hz, 1H), 8.07 (d, *J* = 8.5 Hz, 2H), 7.75 (d, *J* = 1.6 Hz, 1H), 7.70 (d, *J* = 7.8 Hz, 1H), 7.62 (d, *J* = 8.5 Hz, 2H), 7.34 (dd, *J* = 4.8, 7.8 Hz, 1H), 7.23 (s, 1H), 6.72 (d, *J* = 3.4 Hz, 1 H), 6.57 (dd, *J* = 1.6, 3.3 Hz, 1H), 4.40 (d, *J* = 6.0 Hz, 2H). **¹³C NMR** (100 MHz, DMSO-d₆): 165.0, 164.4, 149.7, 148.6, 147.9, 144.8, 136.5, 135.1, 134.9, 132.7, 129.9, 128.4, 126.8, 123.3, 118.3, 114.4, 112.3, 40.3. |
| 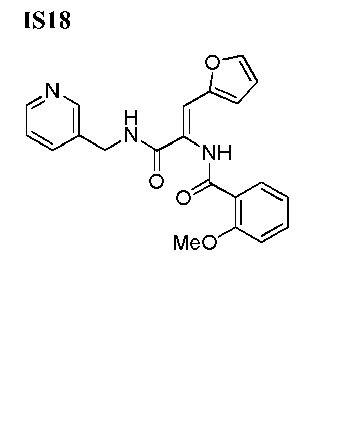 | **¹H NMR** (400 MHz, CDCl₃): 9.96 (s, 1H), 8.48 (d, *J*= 2.1 Hz, 1H), 8.39 (dd, *J* = 1.6, 4.8 Hz, 1H), 8.14 (dd, *J* = 1. 9, 7.9 Hz, 1H), 7.72 (td, *J* = 1.9, 7.8 Hz, 1H), 7.45 (m, 1H), 7.37 (d, *J* = 1.8 Hz, 1H), 7.18 (m, 2H), 7.02 (t, *J* = 7.4 Hz, 1H), 6.96 (d, *J* = 8.3 Hz, 1H), 6.80 (s, 1H), 6.45 (d, *J* = 3.4 Hz, 1H), 6.38 (dd, *J* = 1.8, 3.4 Hz, 1H), 4.50 (d, *J* = 5.9 Hz, 2H), 3.94 (s, 3H); **¹³C NMR** (100 MHz, CDCl₃): 165.7, 164.4, 157.8, 150.4, 149.2, 148.7, 143.7, 135.8, 134.3, 134.0, 132.8, 127.6, 123.6, 121.6, 120.5, 113.7, 113.5, 112.2, 111.5, 56.1, 41.4. LC-MS (ESI): calculated: 377.1; found: 378.2 (M+H)⁺. |
| 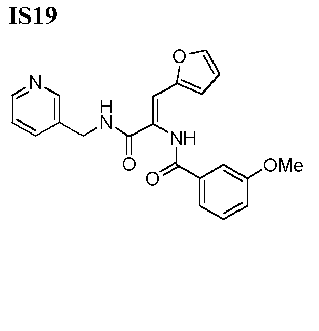 | **¹H NMR** (400 MHz, CDCl₃): 8.84 (s, 1H), 8.43 (d, *J* = 2.3 Hz, 1H), 8.34 (dd, *J* = 1.6, 4.8 Hz, 1H), 7.62 (d, *J* = 7.8 Hz, 1H), 7.48 (t, *J* = 6.0 Hz, 1H), 7.43 (m, 2H), 7.36 (d, *J* = 1.8Hz, 1H), 7.27 (t, *J* = 8.2 Hz, 1H), 7.12 (dd, *J* = 4.8, 7.8 Hz, 1 H), 7.01 (ddd, *J* = 0.8, 2.5, 8.2 Hz, 1H), 6.80 (s, 1H), 6.43 (d, *J*= 3.5 Hz, 1H), 6.36 (dd, *J* = 1.9, 3.5 Hz, 1H), 4.41 (d, *J* = 6.0 Hz, 2H), 3.76 (s, 3H); **¹³C NMR** (100 MHz, CDCl₃): 166.5, 165.6, 160.0, 150.2, 149.0, 148.6, 144.1, 135.8, 134.6, 134.3, 129.8, 127.1, 123.7, 119.7, 118.7, 114.8, 114.7, 112.9, 112.3, 55.6, 41.4. LC-MS (ESI): calculated: 377.1; found: 378.2 (M+H⁺)⁺. |

### IS4, IS5 and IS7

**IS4:** A solution of 3-aminopyridine (94 mg, 1 mmol) and azlactone **3a** (0.24 g, 1 mmol) in CH₃CN (2 mL) was heated under reflux for 3h and allowed to cool to room temperature. The precipitate, was filtered, washed with MeOH and Et₂O and recristalized in Et OH to afford 30 mg of IS4 as a white solid

| | |
|---|---|
| 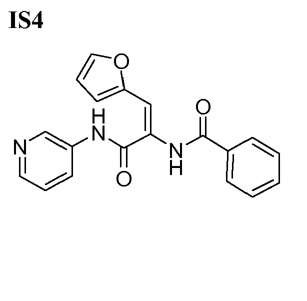 | **¹**H NMR (300 MHz, CDCl₃) : 9.28 (s, 1H), 8.72 (m, 2H), 8.23 (dd, *J* = 1.6, 4.8 Hz, 1H), 8.10 (d, *J* = 8.3 Hz, 1H), 7.93 (d, *J* = 7.2 Hz, 2H), 7.57 (m, 1H), 7.47 (t, *J* = 7.2, 1H), 7.37 (s, 1H), 7.16 (dd, *J* = 4.7, 8.3 Hz, 1H), 6.80 (s, 1H), 6.37 (m, 2H); **¹³C NMR** (100 MHz, DMSO-d₆) : 165.7, 164.0, 149.6, 144.8, 144.4, 141.9, 135.8, 133.6, 131.8, 128.4, 127.9, 127.4, 127.2, 123.4, 117.4, 114.5, 112.4. LC-MS (ESI) : calculated : 333.1 ; found 334.0 (M+H)⁺. |

**IS5:** A solution of 3-aminopyridine (47 mg, 0.5 mmol) and azlactone **3a** (120 mg, 0.5 mmol) in DMF (1 mL) was heated at 130°C for 1h and concentrated in vacuo. The residue was dissolved in AcOEt (50 ml), washed with water, a solution of Na₂CO₃ and brine. Two recristalisations in AcOEt afforded 55 mg of IS5 as a white solid.

| | |
|---|---|
| **IS5** | **¹H NMR** (400 MHz, DMSO-d₆ ): 10.44 (s, 1H), 10.03 (s,1H), 8.44 (dd, *J* = 1.6, 4.9 Hz, 2H), 8.07 (d, *J* = 7.2 Hz, 2H), 7.83 (d, *J* = 1.6 Hz, 1H), 7.72 (dd, *J* = 1.6, 4.9 Hz, 2H), 7.58 (m, 3H), 7.13 (s, 1H), 6.83 (d, *J* = 3.5 Hz, 1H), 6.63 (dd, *J* = 1.9, 3.5 Hz, 1H); **¹³C NMR** (100 MHz, CDCl₃) : 165.7, 164.5, 150.2, 149.5, 145.9, 144.9, 133.4, 131.8, 128.4, 127.9, 127.4, 117.4, 114.7, 113.9, 112.4. LC-MS (ESI): calculated: 333.1; found: 334.0 (M+H⁺)⁺. |
| 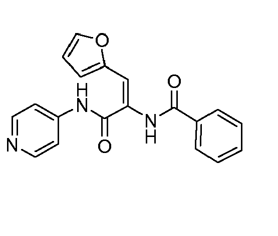 | |

**IS7:** A solution of 2-hydrazinylpyridine (654 mg, 6 mmol) and azlactone **3a** (960 mg, 4 mmol) in CH₂Cl₂/EtOH (16 mL, 1/1) was heated under reflux for 1h and concentrated in vacuo. The residue was purified by flash chromatography (Et₂O followed by EtOAc) and recristalized in Et₂O to afford 0.57 g of IS7 as a white solid.

| | |
|---|---|
| 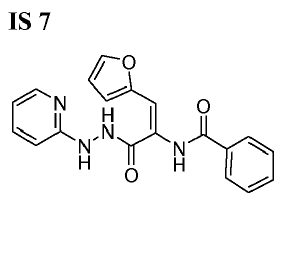 | **¹H NMR** (400 MHz, MeOD-d₄): 9.89 (s, 1H), 9.44 (s, 1H), 8.01 (m, 3H), 7.45 (m, 6H), 7.16 (s, 1H), 6.82 (d, *J* = 8.4Hz, 1H), 6.64 (m, 1H), 6.59 (d, *J* = 3.4 Hz, 1H), 6.41 (dd, *J* = 1.8, 3.5 Hz, 1H); **¹³C NMR** (100 MHz, DMSO-d₆): 165.7, 164.0, 159.2, 149.2, 146.7, 143.3, 136.9, 133.0, 131.0, 127.6, 127.2, 125.0, 116.9, 114.6, 113.6, 111.4, 106.4. LC-MS (ESI): calculated: 348.1; found: 349.0 (M+H⁺)⁺. |

### Cell culture

Coronary endothelial cells (H5V cells) were maintained in DMEM supplemented with 10% heat inactivated Fetal Calf Serum (FCS), glutamine (1mM) under 5% CO2 at 37°C.

### Example 1: Induction of in vitro angiogenesis:

In order to evaluate the potential angiogenic activity of the PKR1 agonists, the test of Growth Factor Reduced Matrigel (GFR Matrigel) 24 well plate was performed. Matrigel leads to the differentiation of many cell types and induces the formation and organization of endothelial cells into capillary tubules. Twenty-four-well culture plates were coated with Matrigel and endothelial cells (H5V) were trypsin-harvested and seeded onto the coated plates at 10⁵ cells per well in the serum free assay medium with PKR1 agonists, IS1 and prokineticin-2, and incubated at 37°C for 24h. Tube formation as two dimensional branched structures were observed using an inverted phase contrast microscope (Zeiss), and the number of branching points were quantified in per well from each sample. Each experiment was repeated at least three times. Images were captured at a magnification of X10 with a digital microscope camera system (Urayama K *et al.*, 2007).

### Results

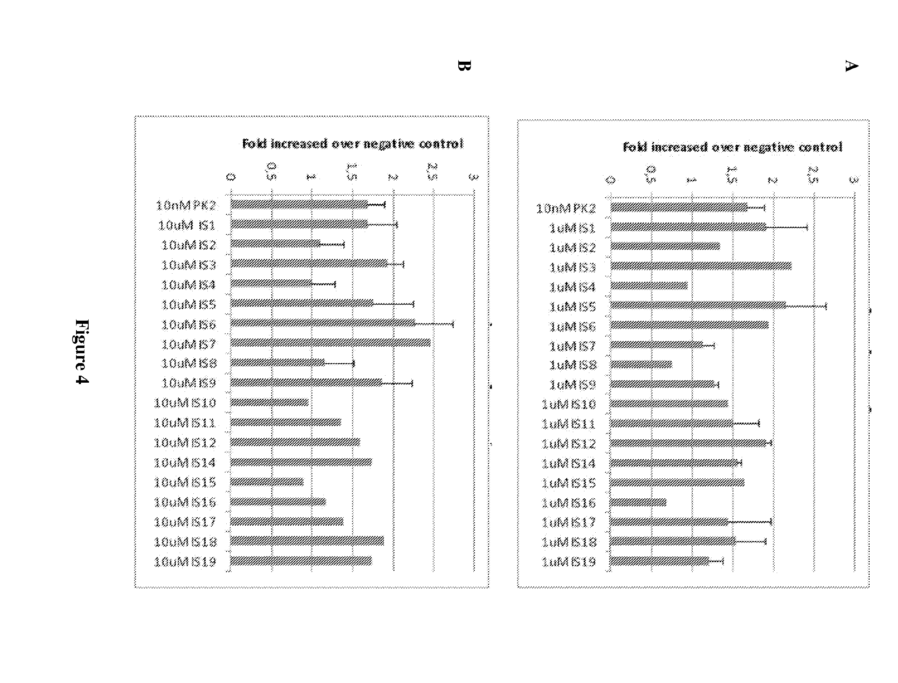

The results are presented in Figures 3 and 4. They demonstrate that the IS compounds (cf. Figure 1) are able to induce *in vitro* angiogenesis as the well known PKR1 agonist, prokineticin-2.

### Example 2: Activation of PKR1 signaling pathway - Measurement of intracellular calcium

CHO-K1 (Chinese Hamster Ovary) cells were infected with a adenovirus carrying PKR1 cDNA. Cells were then stimulated, 48h after infection, with or without IS1 agonists, at 37°C.

Calcium imaging was conducted essentially as previously described (Horinouchi et al., 2007) In brief, Intracellular free calcium [Ca²⁺]i was measured with the fluorescent Ca²⁺ indicator dye Fluo 4-AM, the membrane-permeant acetoxymethyl ester form of Fluo 4 (Invitrogen). Changes in [Ca²⁺]i were determined from variations in the fluorescence intensity of Fluo 4-AM. Briefly, trypsinized CHO-K1-PKR1 cells were seeded onto glass bottom culture dishes (MatTek, MA, USA) for 24 h. Immediately before Ca²⁺ indicator loading, cells were gently washed with HBSS (invitrogen) and then incubated in Fluo 4-AM working solution (Fluo 4-AM 2.5 µM and 2.5 mM probenecid dissolved in standard buffer) for 30 min at 37°C in cell incubator. Afterwards, cells were washed with HBSS including probenecid to remove extracellular Fluo 4-AM. Dishes were then placed on the stage of the laser confocal microscope (Leica). The fluorescence in the cells was excited at a wavelength of 488 nm and a series of images (total exposure time: 300 s) were acquired at 0.5s intervals at emission wavelength 518 nm. Data are presented as the fluorescence intensity of 3 randomly chosen cells within a randomly selected 40 × field. To evaluate the effect of IS1 compound on [Ca²⁺]i in CHO-K-PKR1, the maximum change in fluorescence intensity upon addition of IS1 was measured and normalized to the baseline fluorescence obtained before IS 1 addition.

### Results

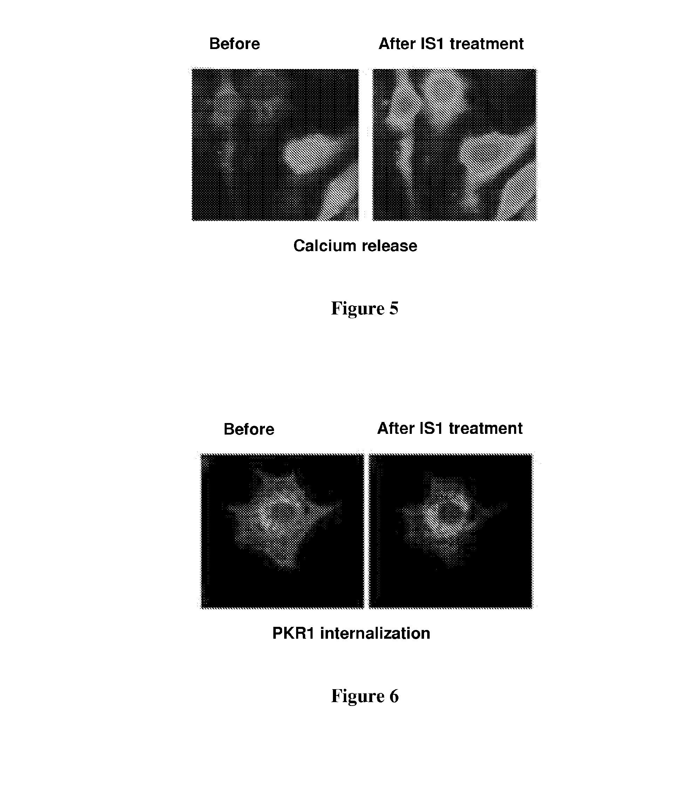

The results are presented in Figure 5. They demonstrate that the IS1 compound is able to activate the PKR1 signalling pathway in cells expressing PKR1.

### Example 3: Specificity of the agonists to PKR1 - Assessment of PKR1 internalization

The capacity of agonist compounds to induce internalization of PKR1 was assessed. Therefore, CHO-K1 cells transfected with a plasmid encoding GFP-PKR1 (Green Fluorescent Protein-PKR1), in the presence of Lipofectamine2000 (Invitrogen), were used. Cells were stimulated, 48h after transfection, with or without agonist compound, at 37°C. GFP is a fluorescent protein fusion (26.9 kDa) which has a major excitation peak at a wavelength of 396 nm and a minor one at 472 nm. Its emission peak is at 504 nm which is in the lower green portion of the visible spectrum. Fluorescence allowed tracking the localization of PKR1 after stimulation by the agonist (membrane or subcellular). Cells were seeded in glass-bottom coated with polyL-lysine and receptor studies were performed in the presence of various PKR1-ligands. Samples were observed under a Leica confocal microscope (SP2 AOBS MP) with objective 63× at 37°C. Images were automatically recorded during 20 min, with increasing time intervals to avoid bleaching effects because of repetitive scanning. Reconstituted videos contained 42 images and lasted 2 second.

### Results

The results are presented in Figure 6. They demonstrate that the IS1 compound specifically interacts with PKR1 and induces its internalization in cells expressing PKR1.

### Conclusion

These experiments demonstrate that the agonists of the invention, and in particular IS compounds (cf. Figure 1) are able to specifically activate the PKR1 signalling pathway inducing the internalization of the receptor and angiogenesis.

### References

Abreu et al. (2010) Neuroendocrinology 91(4):283-90
Cheng et al. (2002) Nature 417, 405-410
Chen et al. (2005) Mol Pharmacol 67:2070-2076
Choke et al. (2009) Eur J Vasc Endovasc Surg 37:305-310.
Dorsch et al. (2005) J. Leukoc. Biol. 78, 426-434
Erlenmeyer, F. (1893). Liebigs Ann. 275: 1-8
Gardiner et al. (2010) Diabetes. 59(2):397-406
Hardelin & Dodé (2008) Sex Dev 2:181-193
Horinouchi et al. (2007) J Pharmacol Sci. 2007; 105:103-111
Kaser et al. (2003) EMBO Rep 4:469-473
LeCouter et al. (2001) Nature 412, 877-884;
LeCouter et al. (2003) Proc. Natl. Acad. Sci.U. S. A. 100, 2685-2690
LeCouter et al. (2004) Proc. Natl. Acad. Sci. U. S. A. 101, 16813-16818 Li et al. (2001) Mol. Pharmacol. 59, 692-698;
Lin et al. (2002) J. Biol. Chem. 277, 19276-19280
Martucci et al. (2006) Br. J. Pharmacol. 147, 225-234
Masuda et al. (2002) Biochem. Biophys. Res. Commun. 293, 396-402
Melchiorri et al. (2001) Eur J Neurosci. 13(9): 1694-702.
Negri, et al. (2005) Br. J. Pharmacol. 146, 625-632
Ng et al. (2005) Science 308, 1923-1927
Parker et al., (2000) Biochim Biophys Acta.149, 369-75.
Plöchl J. (1884) Chem. Ber. 17: 1623
Soga et al. (2002) Biochim. Biophys. Acta. 1579, 173-179
Urayama et al., (2007) FASEB J. 21, 2980-2993
Warner et al., (2006) J Med Genet. 43:e12.1-7

## Claims

1. A pharmaceutical composition comprising a PKR1 agonist of formula (I) 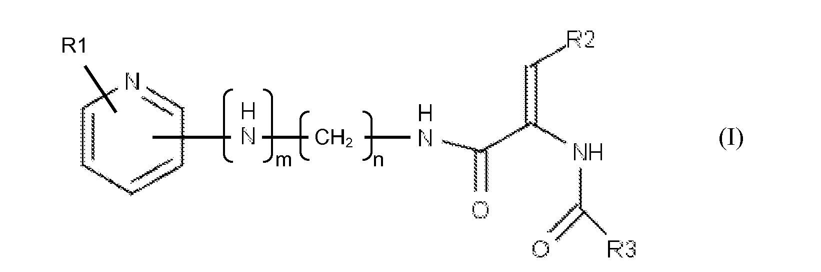 wherein
m is selected from 0 and 1;
n is selected from 0, 1 and 2;
R¹ is selected from the group consisting of a hydrogen atom; a halogen atom; a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; a (C₆-C₁₂)-aryl; a 5 to 7-membered-ring heterocycle; a (C₁-C₆)-alkoxy, a (C₂-C₆)-acyl, a (C₂-C₆)-ester, a (C₁-C₆)-amine, a (C₁-C₆)-amide, a (C₁-C₆)-imine, a (C₁-C₆)-nitrile, a (C₁-C₆)-thioalkyl, a (C₁-C₆)-sulfone and a (C₁-C₆)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a (C₆-C₁₂)-aryl group and a 5 to 7-membered-ring heterocycle, preferably from phenyl and furan, optionally substituted by at least one substituent selected from the group consisting of a (C₁-C₆)-alkyl, a (C₂-C₆)-alkenyl, a (C₂-C₆)-alkynyl, a (C₆-C₁₂)-aryl, a 5 to 7-membered-ring heterocycle, a (C₁-C₆)-alkoxy, a (C₂-C₆)-acyl, a (C₁-C₆)-alcohol, a carboxylic group, a (C₂-C₆)-ester, a (C₁-C₆)-amine, an amino group, a (C₁-C₆)-amide, a (C₁-C₆)-imine, a (C₁-C₆)-nitrile, a hydroxyl, an aldehyde, a (C₁-C₆)-halogenoalkyl, a thiol, a (C₁-C₆)-thioalkyl, a (C₁-C₆)-sulfone, a (C₁-C₆)-sulfoxide group and a halogen atom;
R³ is selected from the group consisting of a (C₁-C₆)-alkyl, a (C₂-C₆)-alkenyl and a (C₂-C₆)-alkynyl and a (C₁-C₆)-amine group, optionally substituted by at least one substituent selected from a hydroxyl, a carboxylic group, an aldehyde, a halogen atom and a thiol; and a (C₆-C₁₂)-aryl and a 5 to 7-membered-ring heterocycle, optionally substituted by at least one substituent selected from the group consisting of a (C₁-C₆)-alkyl, a (C₁-C₆)-alkoxy group and a halogen atom;
or any pharmaceutically acceptable salt thereof.

2. The pharmaceutical composition according to claim 1, wherein R¹ is selected from the group consisting of a hydrogen atom; a halogen atom; a (C₁-C₃)-alkyl, optionally substituted by at least one substituent selected from a hydroxyl, a carboxylic group, an aldehyde, a halogen atom and a thiol; a (C₁-C₃)-alkoxy, a (C₂-C₃)-acyl, a (C₂-C₃)-ester, a (C₁-C₃)-amine, a (C₁-C₃)-amide and a (C₁-C₃)-thioalkyl, group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol.

3. The pharmaceutical composition according to claim 1 or 2, wherein R¹ is selected from the group consisting of a hydrogen atom, a halogen atom, a (C₁-C₃)-alkyl group and a (C₁-C₃)-alkoxy group.

4. The pharmaceutical composition according to any one of claims 1 to 3, wherein R² is selected from the group consisting of a phenyl group and a 5 to 7-membered-ring N-, O-or S-heterocycle, preferably a 5-membered-ring N-, O- or S-heterocycle, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom.

5. The pharmaceutical composition according to any one of claims 1 to 4, wherein R² is selected from the group consisting of a phenyl, a pyrrole, a furan and a thiophene group, optionally substituted by at least one substituent selected from the group consisting of a (C₁-C₃)-alkyl and a (C₁-C₃)-alkoxy group.

6. The pharmaceutical composition according to any one of claims 1 to 4, wherein R³ is selected from the group consisting of a (C₁-C₆)-alkyl and a (C₁-C₆)-amine group, optionally substituted by at least one substituent selected from a hydroxyl, a carboxylic group, an aldehyde, a halogen atom and a thiol; and a phenyl group and a 5 to 7-membered-ring N-, O- or S-heterocycle, optionally substituted by at least one substituent selected from the group consisting of a (C₁-C₃)-alkyl, a (C₁-C₃)-alkoxy group and a halogen atom.

7. The pharmaceutical composition according to any one of claims 1 to 6, wherein R³ is selected from the group consisting of a methyl, an ethyl, a propyl, an isopropyl, a butyl, an isobutyl, a tert-butyl group; and a phenyl group, optionally substituted by at least one substituent selected from the group consisting of a (C₁-C₃)-alkyl group, preferably methyl, a (C₁-C₃)-alkoxy group, preferably methoxy, and a halogen atom, preferably chlorine

8. The pharmaceutical composition according to any one of claims 1 to 7, wherein R¹ is a hydrogen atom; R² is selected from the group consisting of a 5-membered-ring heterocycle and a phenyl group, preferably from a furan, a thiophene and a phenyl group, optionally substituted by a (C₁-C₃)-alkoxy group, preferably a methoxy group; and R³ is selected from the group consisting of a (C₁-C₆)-alkoxy, preferably a tert-butyl group, and a phenyl group, optionally substituted by a substituent selected from the group consisting of a (C₁-C₃)-alkoxy group and a halogen atom, preferably from a methoxy group and chlorine.

9. The pharmaceutical composition of claim 1, wherein the PKR1 agonist is selected from the group consisting of
| Name of agonist | Formula |
|---|---|
| IS1 | 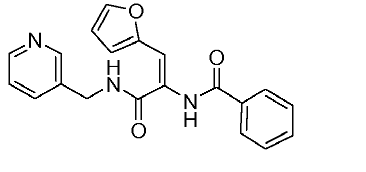 |
| IS2 | 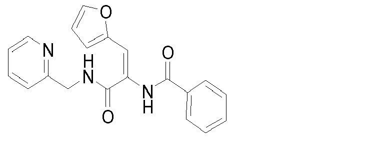 |
| IS3 | 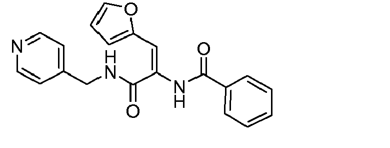 |
| IS4 | 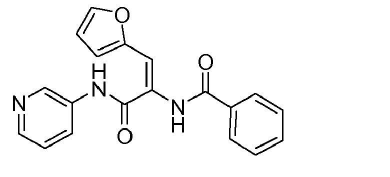 |
| IS5 | 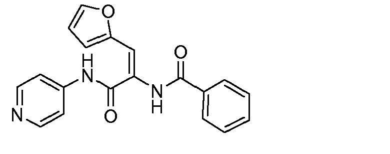 |
| IS6 | 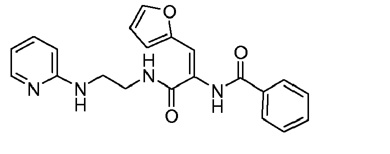 |
| IS7 | 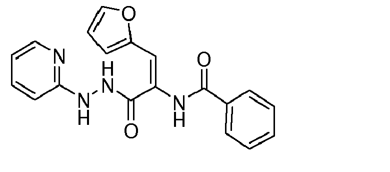 |
| IS8 | 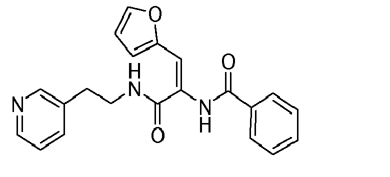 |
| IS9 | 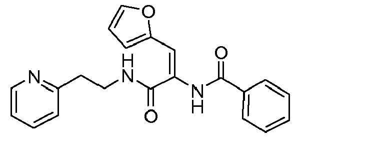 |
| IS10 | 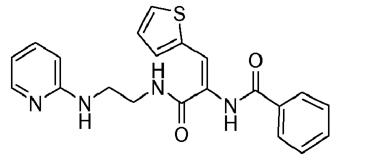 |
| IS11 | 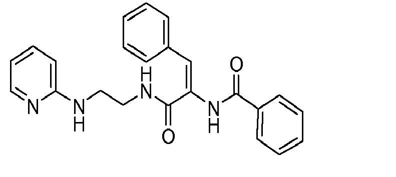 |
| IS12 | 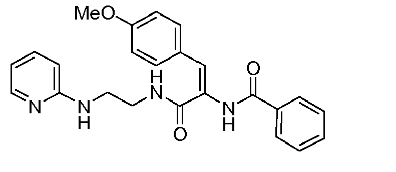 |
| IS14 | 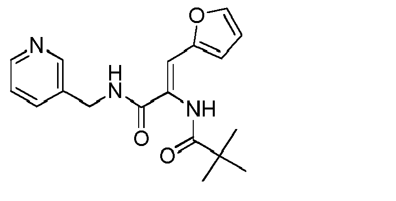 |
| IS15 | 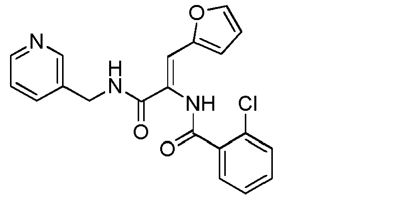 |
| IS16 | 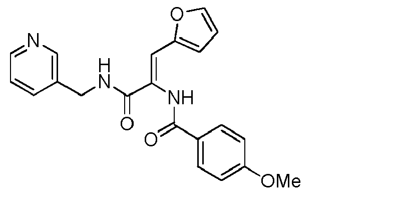 |
| IS17 | 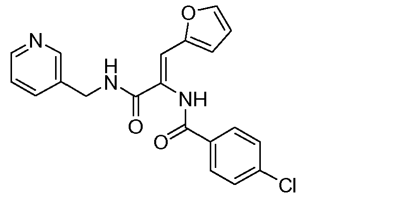 |
| IS18 | 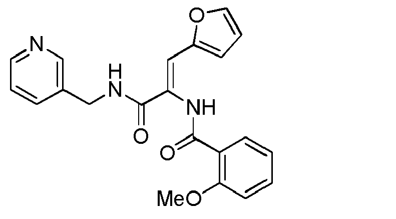 |
| IS19 | 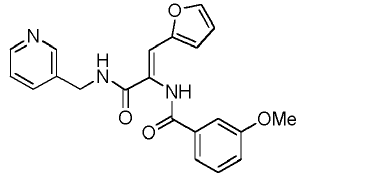 |

10. The pharmaceutical composition according to any one of claims 1 to 9, further comprising one or more other active compounds associated with pharmaceutically acceptable excipients and/or carriers.

11. A pharmaceutical composition according to any one of claims 1 to 10 for use for the prevention or treatment of a disease selected from the group consisting of a vascular disease, a neurodegenerative disease, a disease involving impaired gastrointestinal motility, obesity, Kallmann syndrome, normosmic hypogonadotropic hypogonadism, hyperthyroidism, in particular Familial isolated hyperparathyroidism (FIHP), disturbances of circadian rhythm, sleeping disorders and a pregnancy and placental function-related disorder.

12. The pharmaceutical composition according to claim 11, wherein the disease is a vascular disease selected from the group consisting of myocardial infarction, acute coronary syndrome, ischemic stroke, abdominal aorta aneurysm, cerebral aneurysm, ischemic heart disease, coronary heart disease, peripheral arterial disease, restenosis, angina pectoris and diabetic angiopathy.

13. The pharmaceutical composition according to claim 11, wherein the disease is a neurodegenerative disease selected from the group consisting of Parkinson's disease and other parkinsonian disorders, Alzheimer's disease and other dementing neurodegenerative disorders, amyotrophic lateral sclerosis, multiple sclerosis, Creutzfeldt-Jakob disease, Huntington's disease and neuronal degeneration associated to multiple sclerosis.

14. The pharmaceutical composition according to claim 11, wherein the disease is a disease involving impaired gastrointestinal motility selected from the group consisting of irritable bowel syndrome, diabetic gastroparesis, postoperational ileus, chronic constipation, and gastroesophageal reflux disease, Hirchsprung's disease (congenital colonic aganglionosis) and chronic dyspepsia.

15. A PKR1 agonist as defined in any one of claims 1 to 9, with the proviso that when m is 0, n is 1, R¹ is a hydrogen atom and R² is a furan group, then R³ is not a phenyl group or a phenyl group substituted in para by a methoxy group; and when m is 0, n is 0, R¹ is a hydrogen atom and R² is a furan group, then R³ is not a phenyl group, or any pharmaceutically acceptable salt thereof.
